# EUROPÄISCHE PATENTSCHRIFT

(11) **EP 3 765 466 B1**
(45) Veröffentlichungstag und Bekanntmachung des Hinweises auf die Patenterteilung: **25.05.2022**
(21) Anmeldenummer: 19708558.2
(22) Anmeldetag: 08.03.2019
(51) Int. Cl.: C07D 519/00, A01N 43/90

(54) **KONDENSIERTE BICYCLISCHE HETEROCYCLEN-DERIVATE ALS SCHÄDLINGSBEKÄMPFUNGSMITTEL**
CONDENSED BICYCLIC HETEROCYCLE DERIVATIVES AS PESTICIDES
DÉRIVÉS D'HÉTÉROCYCLÈNE BICYCLIQUES CONDENSÉS EN TANT QUE PESTICIDES

(30) Priorität: 12.03.2018 EP 18161214
(43) Veröffentlichungstag der Anmeldung: 20.01.2021
(73) Patentinhaber: Bayer Aktiengesellschaft, 51373 Leverkusen (DE)
(72) Erfinder: HAGER, Dominik, 40789 Monheim (DE); FISCHER, Rüdiger, 50259 Pulheim (DE); HOFFMEISTER, Laura, 40593 Düsseldorf (DE); WILLOT, Matthieu, 40215 Düsseldorf (DE); ILG, Kerstin, 50670 Köln (DE); LINKA, Marc, 40223 Düsseldorf (DE); GÖRGENS, Ulrich, 40882 Ratingen (DE)
(74) Vertreter: BIP Patents
(86) Internationale Anmeldenummer: PCT/EP2019/055824
(87) Internationale Veröffentlichungsnummer: WO 2019/175046

(56) Entgegenhaltungen:
- EP-A1- 3 257 853
- WO-A1-2017/001311
- WO-A1-2017/093180

## Beschreibung

Die vorliegende Erfindung betrifft neue kondensierte bicyclische Heterocyclen-Derivate der Formel (Ia) oder (Ib), agrochemische Formulierungen enthaltend die Verbindungen gemäß Formel (Ia) oder (Ib) und deren Anwendung als Akarizide und/oder Insektizide zur Bekämpfung tierischer Schädlinge, vor allem von Arthropoden und insbesondere von Insekten und Spinnentieren.

Kondensierte bicyclische Heterocyclen-Derivate mit insektiziden Eigenschaften sind in der Literatur bereits beschrieben, z.B. in WO 2016/162318, WO 2016/129684, WO 2016/023954, WO 2016/142326, WO 2016/169882, WO 2017/093180, WO 2017/061497, WO 2017/001311, WO 2017/001314, WO 2017/084879, WO 2017/089190, WO 2017/133994, WO 2017/155103, WO2018/033455, WO2018/050825, EP 17194731.0, EP18157886.5.

Moderne Insektizide und Akarizide müssen vielen Anforderungen genügen, beispielsweise in Bezug auf Höhe, Dauer und Breite ihrer Wirkung und möglichen Verwendung. Es spielen Fragen der Toxizität, der Nützling- und Bestäuberschonung, der Umwelteigenschaften, der Bodenabbaubarkeit, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Paramenter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen zur Verwendung zur Bekämpfung von tierischen Schädlingen bereitzustellen, durch die das Spektrum der Schädlingsbekämpfungsmittel unter verschiedenen Aspekten ergänzt wird.

Es wurden nun neue kondensierte bicyclische Heterocyclen-Derivate gefunden, welche gegenüber den bereits bekannten Verbindungen Vorteile aufweisen, z.B. seien bessere biologische oder ökologische Eigenschaften, eine optimale Bodenabbaubarkeit, breitere Anwendungsmethoden, eine bessere insektizide, akarizide Wirkung, sowie eine gute Verträglichkeit gegenüber Nutzpflanzen beispielhaft genannt. Die kondensierten bicyclischen Heterocyclen-Derivate können in Kombination mit weiteren Mitteln zur Verbesserung der Wirksamkeit insbesondere gegen schwierig zu bekämpfende Insekten eingesetzt werden.

### Gegenstand der vorliegenden Erfindung sind daher neue

Verbindungen der Formel (Ia) oder (Ib) (Ausgestaltung 5) 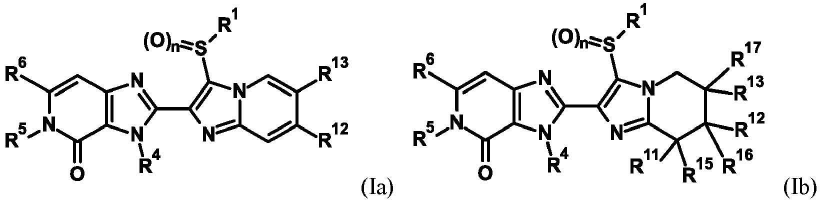 wobei
- R¹: für (C₁-C₄)Alkyl steht,
- R¹²: für Wasserstoff, (C₁-C₄)Alkyl, Halogen, (C₃-C₆)Cycloalkyl-(C₁-C₆)halogenalkyl, (C₃-C₆)Cycloalkylsulfonyl, (C₃-C₆)Cycloalkylcarbonyl, (C₁-C₄)Halogenalkyl oder gegebenenfalls einfach durch Cyano substituiertes (C₃-C₆)Cycloalkyl steht,
- R¹¹, R¹³, R¹⁶: unabhängig voneinander für Wasserstoff oder (C₁-C₄)Alkyl stehen,
- R¹⁵, R¹⁷: für Wasserstoff stehen, wobei maximal zwei der Reste R¹¹, R¹², R¹³ oder R¹⁶ für einen Substituenten ungleich Wasserstoff stehen und
- R⁴, R⁵: unabhängig voneinander für (C₁-C₄)Alkyl stehen,
- R⁶: für (C₁-C₄)Halogenalkyl steht und
- n: für 2 steht.

Weiterhin wurde gefunden, dass die Verbindungen der Formel (Ia) oder (Ib) eine sehr gute Wirksamkeit als Schädlingsbekämpfungsmittel, vorzugsweise als Insektizide und/oder Akarizide aufweisen, eine optimale Bodenabbaubarkeit zeigen und darüber hinaus in der Regel insbesondere gegenüber Kulturpflanzen sehr gut pflanzenverträglich sind.

Die erfindungsgemäßen Verbindungen sind durch die Formel (Ia) oder (Ib) allgemein definiert. Bevorzugte Substituenten bzw. Bereiche der in der oben und nachstehend erwähnten Formeln aufgeführten Reste werden im Folgenden erläutert:

### Ausgestaltung 6-1

Verbindungen der Formel (Ia) oder (Ib) 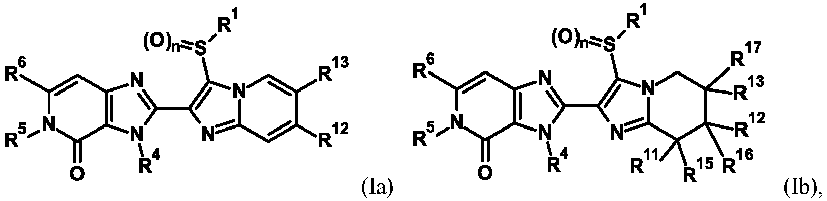 wobei
- R¹: insbesondere für Ethyl steht,
- R¹²: insbesondere für Wasserstoff, Cyanocyclopropyl oder Trifluormethyl steht,
- R¹³: insbesondere für Wasserstoff oder Methyl steht,
- R¹¹, R¹⁵, R¹⁶, R¹⁷: insbesondere für Wasserstoff stehen,
- R⁴, R⁵: insbesondere für Methyl stehen,
- R⁶: insbesondere für Trifluormethyl steht und
- n: insbesondere für 2 steht.

### Ausgestaltung 6-2

Verbindungen der Formel (Ia) oder (Ib) 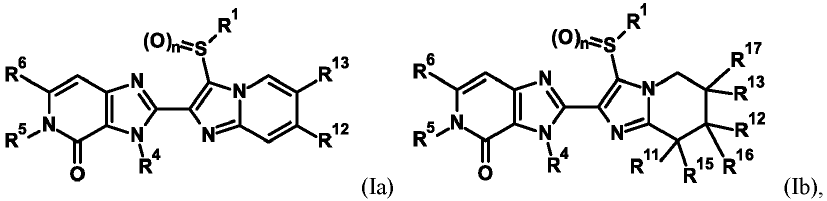 wobei
- R¹: insbesondere für Ethyl steht,
- R¹²: insbesondere für Wasserstoff, Cyanocyclopropyl, Methyl, Trifluormethyl, Cyclopropylcarbonyl, Cyclopropylsulfonyl oder Cyclopropyldifluormethyl steht,
- R¹¹, R¹³, R¹⁶: unabhängig voneinander insbesondere für Wasserstoff oder Methyl stehen,
- R¹⁵, R¹⁷: insbesondere für Wasserstoff stehen,
wobei maximal zwei der Reste R¹¹, R¹², R¹³ oder R¹⁶ für einen Substituenten ungleich Wasserstoff stehen und
- R⁴, R⁵: insbesondere für Methyl stehen,
- R⁶: insbesondere für Trifluormethyl steht und
- n: insbesondere für 2 steht.

Im Folgenden betreffen die Ausführungen zu den Verbindungen der Formel (Ia) oder (Ib) selbstverständlich auch die Verbindungen der Formenl (Ia') und (Ib'), welche von Formel (Ia) und (Ib) umfasst sind.

Im Folgenden steht der Begriff *Ausgestaltung 6* für *Ausgestaltung 6-1 oder Ausgestaltung 6-2.*

In einer hervorgehobenen Ausführungsform betrifft die Erfindung Verbindungen der Formel (Ia) oder (Ib), wobei
- R¹: für Ethyl steht und
n, R⁴, R⁵, R⁶, R¹¹, R¹², R¹³, R¹⁴, R¹⁵, R¹⁶ und R¹⁷ Ausgestaltung (5) beschriebenen Bedeutungen haben.

In einer hervorgehobenen Ausführungsform betrifft die Erfindung Verbindungen der Formel (Ia) oder (Ib), wobei
R⁴ für Methyl steht und
n, R¹, R⁵, R⁶, R¹¹, R¹², R¹³, R¹⁴, R¹⁵, R¹⁶ und R¹⁷ die in Ausgestaltung (5) beschriebenen Bedeutungen haben.

In einer hervorgehobenen Ausführungsform betrifft die Erfindung Verbindungen der Formel (Ia) oder (Ib), wobei
- R⁵: für Methyl steht und
n, R¹, R⁴, R⁶, R¹¹, R¹², R¹³, R¹⁴, R¹⁵, R¹⁶ undR¹⁷ die in Ausgestaltung (5) beschriebenen Bedeutungen haben.

In einer hervorgehobenen Ausführungsform betrifft die Erfindung Verbindungen der Formel (Ia) oder (Ib), wobei
- R⁶: für Trifluormethyl steht und
n, R¹, R⁴, R⁵, R¹¹, R¹², R¹³, R¹⁴, R¹⁵, R¹⁶ und R¹⁷ die in Ausgestaltung (5) beschriebenen Bedeutungen haben.

In einer ganz besonders bevorzugten Ausführungsform betrifft die Erfindung Verbindungen der Formel (Ia) oder (Ib), wobei
- R¹: für Ethyl steht,
- R⁴: für Methyl steht,
- R⁵: für Methyl steht,
- R⁶: für Trifluormethyl steht und
n, R¹¹, R¹², R¹³, R¹⁴, R¹⁵, R¹⁶ und R¹⁷ die in Ausgestaltung (5) beschriebenen Bedeutungen haben.

In einer insbesonders bevorzugten Ausführungsform betrifft die Erfindung Verbindungen der Formel (Ia) oder (Ib), wobei
- R¹²: für Wasserstoff, Cyanocyclopropyl, Methyl, Trifluormethyl, Cyclopropylcarbonyl, Cyclopropylsulfonyl oder Cyclopropyldifluormethyl steht,
- R¹¹, R¹³, R¹⁶: unabhängig voneinander für Wasserstoff oder Methyl stehen,
- R¹⁴, R¹⁸, R¹⁵ und R¹⁷: für Wasserstoff stehen,
wobei maximal zwei der Reste R¹¹, R¹², R¹³ oder R¹⁶ für einen Substituenten ungleich Wasserstoff stehen und
n, R¹, R⁴, R⁵ und R⁶ die in Ausgestaltung (5) beschriebenen Bedeutungen haben.

Besonders bevorzugt ergeben sich für die Formeln (Ia) oder (Ib) die folgenden Strukturen (Ia') oder (Ib'), 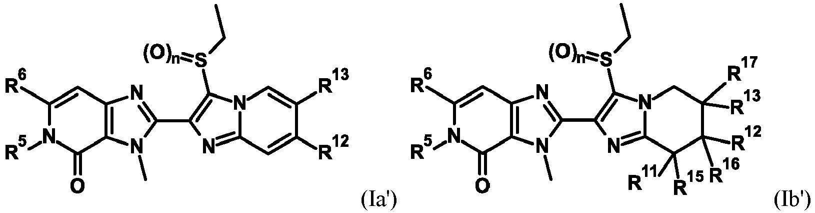 wobei R⁵, R⁶, R¹¹, R¹², R¹³, R¹⁵, R¹⁶, R¹⁷ und n die oben beschriebenen, insbesondere die in Ausgestaltung (5) oder Ausgestaltung (6) beschriebenen Bedeutungen haben.

In einer bevorzugten Ausführungsform der Erfindung steht in den Formeln (Ia) und (Ia') R¹³ für Wasserstoff.

Weiterhin bevorzugt sind Verbindungen der Formel I(a), 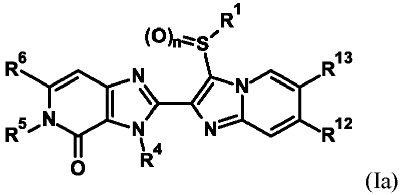 wobei R¹, R⁴, R⁵, R⁶, R¹², R¹³ und n die in Ausgestaltung (5) oder Ausgestaltung (6) beschriebenen Bedeutungen haben.

Besonders bevorzugt sind Verbindungen der Formel I(a), wobei gilt,
- R¹: steht für (C₁-C₄)Alkyl,
- R¹²: steht für Wasserstoff, (C₁-C₄)Alkyl, Halogen, (C₃-C₆)Cycloalkyl-(C₁-C₆)halogenalkyl, (C₃-C₆)Cycloalkylsulfonyl, (C₃-C₆)Cycloalkylcarbonyl, (C₁-C₄)Halogenalkyl oder gegebenenfalls einfach durch Cyano substituiertes (C₃-C₆)Cycloalkyl,
- R¹¹,: steht für Wasserstoff,
- R⁴, R⁵: stehen unabhängig voneinander f für (C₁-C₄)Alkyl,
- R⁶: steht für (C₁-C₄)Halogenalkyl und
- n: steht für 2.

Ganz besonders bevorzugt sind Verbindungen der Formel I(a), wobei gilt,
- R¹: steht für Ethyl,
- R¹²: steht für Wasserstoff, Cyanocyclopropyl, Methyl, Trifluormethyl, Cyclopropylcarbonyl, Cyclopropylsulfonyl oder Cyclopropyldifluormethyl,
- R¹¹,: steht für Wasserstoff,
- R⁴, R⁵: stehen für Methyl,
- R⁶: steht für Trifluormethyl und
- n: steht für 2.

Weiterhin bevorzugt sind Verbindungen der Formel I(b), 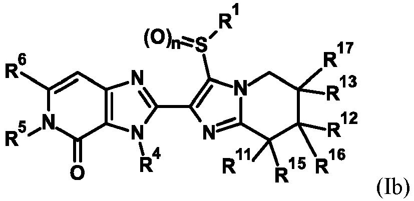 wobei R¹, R⁴, R⁵, R⁶, R¹¹, R¹², R¹³, R¹⁵, R¹⁶, R¹⁷ und n die in (5) oder Ausgestaltung (6) beschriebenen Bedeutungen haben.

Besonders bevorzugt sind Verbindungen der Formel I(b), wobei gilt,
- R¹: steht für (C₁-C₄)Alkyl,
- R¹²: steht für Wasserstoff, (C₁-C₄)Alkyl oder (C₁-C₄)Halogenalkyl,
- R¹¹, R¹³, R¹⁶: stehen unabhängig voneinander für Wasserstoff oder (C₁-C₄)Alkyl,
- R¹⁵, R¹⁷: stehen für Wasserstoff,
wobei maximal zwei der Reste R¹¹, R¹², R¹³ oder R¹⁶ für einen Substituenten ungleich Wasserstoff stehen und
- R⁴, R⁵: stehen unabhängig voneinander für (C₁-C₄)Alkyl,
- R⁶: steht für (C₁-C₄)Halogenalkyl und
- n: steht für 2.

Ganz besonders bevorzugt sind Verbindungen der Formel I(b), wobei gilt,
- R¹: steht für Ethyl,
- R¹²: steht für Wasserstoff, Methyl oder Trifluormethyl,
- R¹¹, R¹³, R¹⁶: stehen unabhängig voneinander für Wasserstoff oder Methyl,
- R¹⁵, R¹⁷: stehen für Wasserstoff,
wobei maximal zwei der Reste R¹¹, R¹², R¹³ oder R¹⁶ für einen Substituenten ungleich Wasserstoff stehen und
- R⁴, R⁵: stehen für Methyl,
- R⁶: stehen für Trifluormethyl und
- n: steht für 2.

Definitionsgemäß ist, sofern nichts anderes angegeben ist, Halogen ausgewählt aus der Reihe Fluor, Chlor, Brom und Iod, bevorzugt wiederum aus der Reihe Fluor, Chlor und Brom.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Sofern nicht an anderer Stelle anders definiert, wird unter dem Begriff "Aryl" erfindungsgemäß ein aromatischer Rest mit 6 bis 14 Kohlenstoffatomen, vorzugsweise Phenyl, Naphthyl, Anthryl oder Phenanthrenyl, besonders bevorzugt Phenyl, verstanden.

Sofern nicht an anderer Stelle anders definiert, wird unter dem Begriff "Arylalkyl" eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Sofern nicht an anderer Stelle anders definiert, bedeutet "Hetaryl" eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5, 6, 7 oder 8 C-Atome und ist ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Imidazopyridinyl und Indolizinyl.

Sofern nicht an anderer Stelle anders definiert, bedeutet "Heterocyclyl" einen monocyclischen, gesättigten oder teilgesättigten 4-, 5-, 6- oder 7-gliedrigen Ring aus C-Atomen und mindestens einem Heteroatom im Ring. Bevorzugt enthält die Heterocyclyl-Gruppe 3, 4, 5 oder 6 C-Atome und 1 oder 2 Heteroatome aus der Reihe Sauerstoff, Schwefel oder Stickstoff. Beispiele für Heterocyclyl sind Azetidinyl, Azolidinyl, Azinanyl, Oxetanyl, Oxolanyl, Oxanyl, Dioxanyl, Thiethanyl, Thiolanyl, Thianyl, Tetrahydrofuryl.

Die Verbindungen der Formel (Ia) oder (Ib) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die Verbindungen der Formel (Ia) oder (Ib) können auch als Salze, insbesondere Säureadditionssalze und Metallsalzkomplexe, vorliegen. Die Verbindungen der Formel (Ia) oder (Ib) und deren Säureadditionssalze und Metallsalzkomplexe besitzen gute Wirksamkeit, insbesondere zur Bekämpfung von tierischen Schädlingen.

Als geeignete Salze der Verbindungen der allgemeinen Formel (Ia) oder (Ib) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (Ia) oder (Ib), in welchen eine Kombination der vorstehend als bevorzugt aufgeführten Bedeutungen vorliegt.

Erfindungsgemäß besonders bevorzugt verwendet werden Verbindungen der Formel (Ia) oder (Ib), in welchen eine Kombination der vorstehend als besonders bevorzugt aufgeführten Bedeutungen vorliegt.

Erfindungsgemäß ganz besonders bevorzugt verwendet werden Verbindungen der Formel (Ia) oder (Ib), in welchen eine Kombination der vorstehend als ganz besonders bevorzugt aufgeführten Bedeutungen vorliegt.

Erfindungsgemäß hervorgehoben verwendet werden Verbindungen der Formel (Ia) oder (Ib), in welchen eine Kombination der vorstehend als hervorgehoben aufgeführten Bedeutungen vorliegt.

Erfindungsgemäß insbesonders verwendet werden Verbindungen der Formel (Ia) oder (Ib), in welchen eine Kombination der vorstehend als insbesonders aufgeführten Bedeutungen vorliegt.

Die erfindungsgemäßen Verbindungen der Formel (Ia) oder (Ib) können durch die in den folgenden Schemata dargestellten Verfahren erhalten werden:

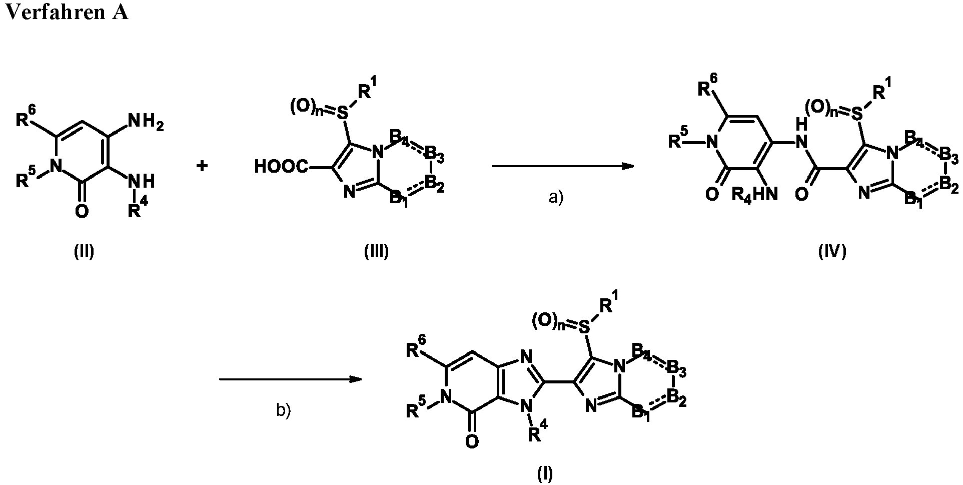

Die Variablen B₁, B₂, B₃, B₄, R¹, R⁴, R⁵, R⁶ und n sind dabei wie oben gemäß Formel (Ia) oder (Ib), insbesondere in den Ausgestaltungen 5 bis 6 beschrieben, definiert.

### Schritt a)

Die Verbindungen der Formel (IV) können durch die Umsetzung von Verbindungen der Formel (II) mit Carbonsäuren der Formel (III) in Gegenwart eines Kondensationsmittels bzw. einer Base hergestellt werden.

Verbindungen der Formel (II) können nach den in WO2016/023954 beschriebenen Verfahren hergestellt werden. Carbonsäuren der Formel (III) können nach den in WO2016/162318 publizierten Verfahren hergestellt werden. Weitere Verfahren zur Herstellung von Carbonsäuren (III) sind in Verfahren B und C beschrieben.

Die Umsetzung der Verbindungen der Formel (II) mit Carbonsäuren der Formel (III) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Kondensationsmittel sind beispielsweise Carbodiimide wie 1-(3-Dimethylaminopropyl)-3-ethylcarbodiimid hydrochlorid (EDCI), 1,3-Dicyclohexylcarbodiimid, Thionylchlorid, oder Oxalylchlorid.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

### Schritt b)

Die Verbindungen der Formel (Ia) oder (Ib) lassen sich herstellen durch Kondensation der Verbindungen der Formel (IV) z.B. analog der in WO2009/131237, WO2010/125985, WO2011/043404, WO2011/040629, WO2012/086848, WO2013/018928, WO2015/000715 oder WO 2015/121136 beschriebenen Verfahren.

Die Umsetzung zu Verbindungen der Formel (IV) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Die Reaktion lässt sich durchführen in Gegenwart eines Kondensationsmittels, einer Säure, einer Base oder eines Chlorierungsmittels.

Beispiele für geeignete Kondensationsmittel sind Carbodiimide wie 1-(3-Dimethylaminopropyl)-3-ethylcarbodiimid hydrochlorid (EDCI) oder 1,3-Dicyclohexylcarbodiimid; Anhydride wie Essigsäureanhydrid, Trifluoressigsäureanhydrid; eine Mischung aus Triphenylphosphin, einer Base und Tetrachlorkohlenstoff oder eine Mischung aus Triphenylphosphin und einem Azodiester wie z.B. Diethylazodicarbonsäure.

Beispiele für geeignete Säuren, die in der beschriebenen Reaktion eingesetzt werden können, sind Sulfonsäuren wie para-Toluolsulfonsäure; Carbonsäuren wie Essigsäure oder Polyphosphorsäuren.

Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin; anorganische Basen wie Kaliumphosphat, Kaliumcarbonat und Natriumhydrid.

Ein Beispiel für ein geeignetes Chlorierungsmittel ist Phosphoroxychlorid.

Die Reaktion kann im Vakuum, bei Normaldruck oder unter Überdruck und bei Temperaturen von 0 °C bis 200 °C durchgeführt werden.

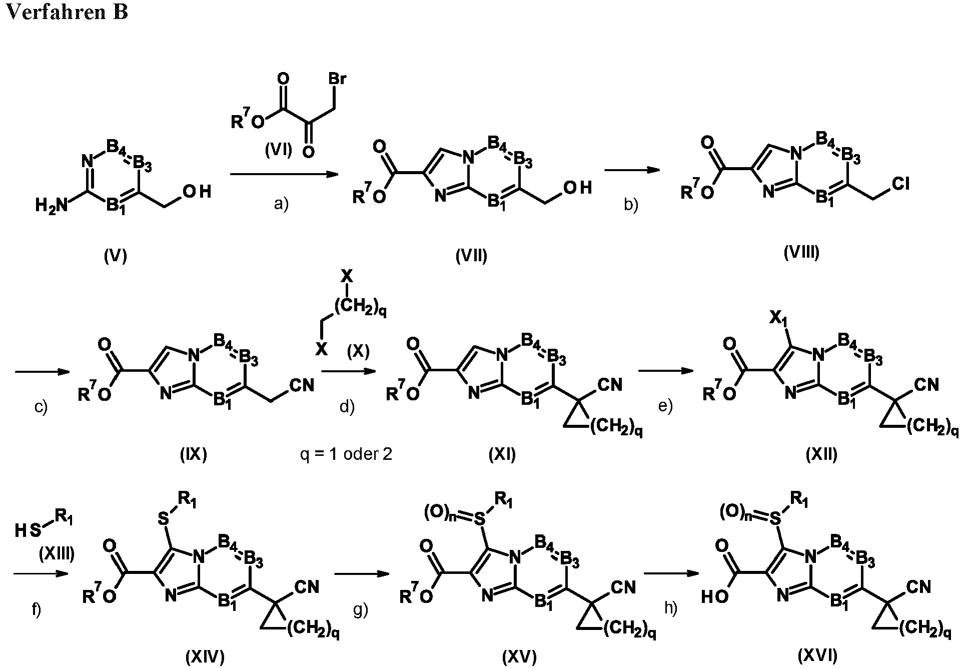

Die Variablen B₁, B₃, B₄, R¹ und n sind dabei wie oben gemäß Formel (Ia) oder (Ib), insbesondere in den Ausgestaltungen 5 bis 6 beschrieben, definiert. X steht dabei für Cl, Br oder I, R⁷ für (C₁-C₄)Alkyl und q für 1 oder 2.

### Schritt a)

Die Verbindungen der Formel (VII), können in Analogie zu den in European Journal of Medicinal Chemistry, 29 (1994) 279-286; WO2006/71752; WO2012/80232; Journal of Medicinal Chemistry, 57 (2014), 4196-4212; WO2012/143599; WO2015/48245 and WO2006/18725 beschriebenen Verfahren durch Umsetzung von Verbindungen der Formel (V) mit einer geeigneten Carbonylverbindung, beispielsweise einem Brompyruvat-Derivat der Formel (VI), bei Raumtemperatur oder unter thermischen Bedingungen in einem geeigneten Lösungsmittel wie beispielsweise Ethanol, Tetrahydrofuran, Acetonitril oder Dimethylformamid hergestellt werden.

Die Brompyruvat-Derivate der Formel (VI) sind kommerziell erhältlich. Die Verbindungen der Formel (V) sind entweder kommerziell erhältlich oder können nach bekannten Methoden hergestellt werden, beispielsweise analog der in Chemical Communications, 44 (2010), 925-927; Journal of the American Chemical Society, 68 (1946), 453-457; WO2009/29625; Journal ofthe American Chemical Society, 137 (2015), 8388-8391; Journal of Medicinal Chemistry, 57 (2014), 4196-4212, Helvetica Chimica Acta, 55 (1972), 565-568 und Synthesis, 9 (1985), 884-886 beschriebenen Verfahren.

### Schritt b)

Die Verbindungen der Formel (VIII) lassen sich aus Verbindungen der Formel (VII) herstellen, z.B. analog der in WO2008/36216, WO2004/22561, WO2006/23707, WO2006/133006, WO2014/60375, US2004/23981 oder EP3018125 beschriebenen Verfahren.

Die Umsetzung zu Verbindungen der Formel (VIII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Die Reaktion lässt sich durchführen in Gegenwart eines Chlorierungsmittels und gegebenenfalls einer Base.

Beispiele für geeignete Chlorierungsmittel sind Thionylchlorid, Methansulfonsäurechlorid, oder Phosphorylchlorid.

Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin; anorganische Basen wie Kaliumphosphat, Kaliumcarbonat und Natriumhydroxid.

Die Reaktion kann im Vakuum, bei Normaldruck oder unter Überdruck und bei Temperaturen von 0 °C bis 200 °C durchgeführt werden.

### Schritt c)

Die Verbindungen der Formel (IX) lassen sich herstellen durch Substitution aus Verbindungen der Formel (VIII), z.B. analog der in US2014/57914, EP2036905, J. Agric. Food Chem. 2017, 65, 1272-1280, WO2009/114180, oder Tetrahedron 2005, 6115 beschriebenen Verfahren.

Die Umsetzung zu Verbindungen der Formel (IX) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden polare Lösungsmittel wie beispielsweise Dimethylsulfoxid oder N,N-Dimethylformamid oder Acetonitril.

Die Reaktion lässt sich durchführen in Gegenwart eines Cyanierungsmittels.

Beispiele für geeignete Cyanierungsmittel sind Natriumcyanid oder Kaliumcyanid.

Die Reaktion kann im Vakuum, bei Normaldruck oder unter Überdruck und bei Temperaturen von 0 °C bis 200 °C durchgeführt werden.

### Schritt d)

Verbindungen der Formel (XI) können hergestellt werden durch Umsetzung der Verbindungen der Formel (IX) mit Verbindungen der Formel (X) in Gegenwart einer Base, beispielsweise nach den in WO2016/041819 beschriebenen Verfahren.

Die Verbindungen der Formel (X) sind kommerziell erhältlich,

Die Umsetzung zu Verbindungen der Formel (IX) erfolgt in der Regel in einem Lösungsmittel. Bevorzugt werden halogenierte Kohlenwasserstoffe wie beispielsweise Dichlormethan, Chloroform, Tetrachlorkohlenstoff, 1,2-Dichlorethan oder Chlorbenzol, aprotische polare Lösungsmittel wie beispielsweise Aceton, N,N-Dimethylformamid, N-Methylpyrrolidon, Dimethylsulfoxid, Nitrile wie beispielsweise Acetonitril, oder Ester wie beispielsweise Essigsäureethylester.

Beispiele für geeignete Basen sind stickstoffhaltige Heterocyclen wie Pyridin, 1,8-Diazabicyclo[5.4.0]-7-undecen (DBU); tertiäre Amine wie Triethylamin und N,N-Diisopropylethylamin; anorganische Basen wie Kaliumphosphat, Cäsiumcarbonat, Kaliumcarbonat und Natriumhydrid.

Die Reaktion kann im Vakuum, bei Normaldruck oder unter Überdruck und bei Temperaturen von 0°C bis 200 °C durchgeführt werden.

### Schritt e)

Verbindungen der Formel (XII) können nach bekannten Methoden aus Verbindungen der Formel (XI) über eine Halogenierung hergestellt werden in Analogie zu den in WO2009/23179, WO2010/91411, WO2011/41713 und Bioorganic and Medicinal Chemistry Letters, 22 (2012), 3460-3466 beschriebenen Verfahren, beispielsweise mit N-Chlorsuccinimid als Halogenierungsmittel in Dimethylformamid als Lösungsmittel.

### Schritt f)

Die Verbindungen der Formel (XIV) lassen sich herstellen durch Umsetzung der Verbindungen der Formel (XII) mit den Verbindungen der Formel (XIII) in Gegenwart einer Base.

Mercaptanderivate der Formel (XIII) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

Die Umsetzung zu Verbindung der Formel (XII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat. Weitere geeignete Basen sind Alkalimetallhydride wie z.B. Natriumhydrid.

### Schritt g)

Die Verbindungen der Formel (XV) lassen sich herstellen durch Oxidation der Verbindungen der Formel (XIV). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20 °C bis 120 °C durchgeführt werden.

### Schritt h)

Die Ester der Formel (XV) können unter Verwendung von Standardmethoden, vgl. DE 2221647 und WO2011/41713, in die Säure der Formel (XVI) überführt werden, beispielsweise mit einem Alkalihydroxid als Base wie Natriumhydroxid oder Lithiumhydroxid in einem Alkohol als Lösungsmittel wie z.B. Ethanol oder einem Gemisch aus Tetrahydrofuran und Wasser.

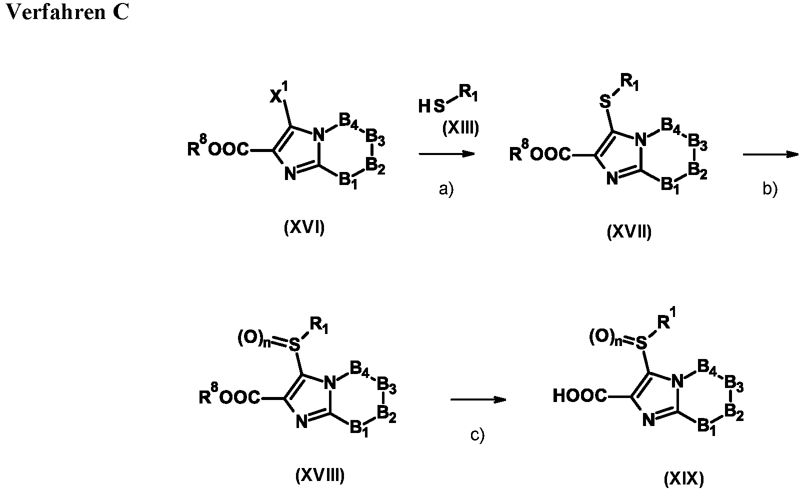

Die Variablen B₁, B₂, B₃, B₄, R¹ und n sind dabei wie oben gemäß Formel (Ia) oder (Ib), insbesondere in den Ausgestaltungen 5 bis 6 beschrieben, definiert. X¹ steht dabei für Cl, Br oder I und R⁸ für (C₁-C₄)Alkyl.

### Schritt a)

Die Verbindungen der Formel (XVII) lassen sich herstellen durch Umsetzung der Verbindungen der Formel (XVI) mit den Verbindungen der Formel (XIII) in Gegenwart einer Base.

Carbonsäureester der Formel (XVI) sind entweder kommerziell erhältlich oder können nach bekannten Methoden hergestellt werden, beispielsweise analog der in WO2018/033455 beschriebenen Verfahren

Mercaptanderivate der Formel (XIII) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

Die Umsetzung zu Verbindung der Formel (XVII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat. Weitere geeignete Basen sind Alkalimetallhydride wie z.B. Natriumhydrid.

### Schritt b)

Die Verbindungen der Formel (XVIII) lassen sich herstellen durch Oxidation der Verbindungen der Formel (XVII). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20 °C bis 120 °C durchgeführt werden.

### Schritt c)

Die Ester der Formel (XVIII) können unter Verwendung von Standardmethoden, vgl. DE 2221647 und WO2011/41713, in die Säure der Formel (XIX) überführt werden, beispielsweise mit einem Alkalihydroxid als Base wie Natriumhydroxid oder Lithiumhydroxid in einem Alkohol als Lösungsmittel wie z.B. Ethanol oder einem Gemisch aus Tetrahydrofuran und Wasser.

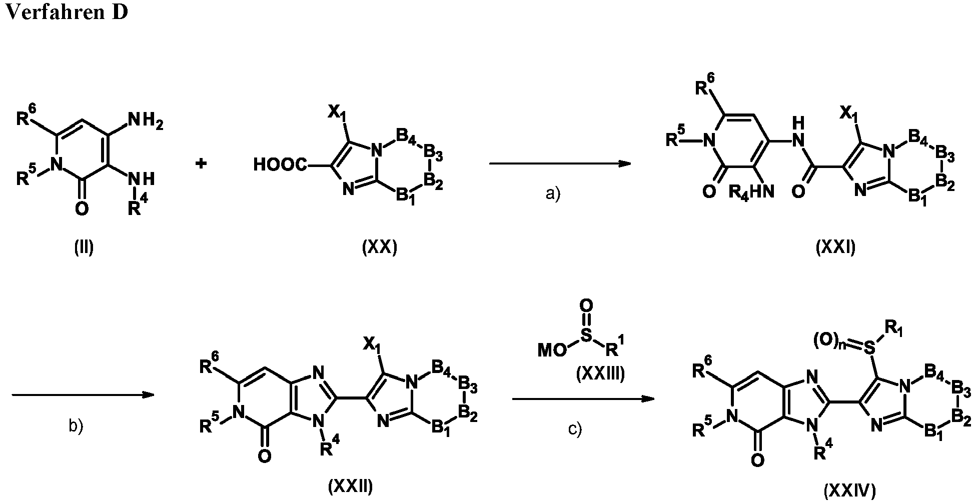

Die Variablen B₁, B₂, B₃, B₄, R¹, R⁴, R⁵, R⁶ und n sind dabei wie oben gemäß Formel (Ia) oder (Ib), insbesondere in den Ausgestaltungen 5 bis 6 beschrieben, definiert. X¹ steht dabei für Cl, Br oder I und M steht dabei für Na, K oder Li.

### Schritt a)

Die Verbindungen der Formel (XXII) können durch die Umsetzung von Verbindungen der Formel (II) mit Carbonsäuren der Formel (XX) in Gegenwart eines Kondensationsmittels bzw. einer Base hergestellt werden.

Verbindungen der Formel (II) können nach den in WO2016/023954 beschriebenen Verfahren hergestellt werden. Carbonsäuren der Formel (XX) sind entweder kommerziell erhältlich oder können nach bekannten Methoden hergestellt werden, beispielsweise analog der in WO2018/033455 beschriebenen Verfahren.

Die Umsetzung der Verbindungen der Formel (II) mit Carbonsäuren der Formel (XX) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Kondensationsmittel sind beispielsweise Carbodiimide wie 1-(3-Dimethylaminopropyl)-3-ethylcarbodiimid hydrochlorid (EDCI), 1,3-Dicyclohexylcarbodiimid, Thionylchlorid, oder Oxalylchlorid.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

### Schritt b)

Die Verbindungen der Formel (XXII) lassen sich herstellen durch Kondensation der Verbindungen der Formel (XI) z.B. analog der in WO2009/131237, WO2010/125985, WO2011/043404, WO2011/040629, WO2012/086848, WO2013/018928, WO2015/000715 oder WO 2015/121136 beschriebenen Verfahren.

Die Umsetzung zu Verbindungen der Formel (XXII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Die Reaktion lässt sich durchführen in Gegenwart eines Kondensationsmittels, einer Säure, einer Base oder eines Chlorierungsmittels.

Beispiele für geeignete Kondensationsmittel sind Carbodiimide wie 1-(3-Dimethylaminopropyl)-3-ethylcarbodiimid hydrochlorid (EDCI) oder 1,3-Dicyclohexylcarbodiimid; Anhydride wie Essigsäureanhydrid, Trifluoressigsäureanhydrid; eine Mischung aus Triphenylphosphin, einer Base und Tetrachlorkohlenstoff oder eine Mischung aus Triphenylphosphin und einem Azodiester wie z.B. Diethylazodicarbonsäure.

Beispiele für geeignete Säuren, die in der beschriebenen Reaktion eingesetzt werden können, sind Sulfonsäuren wie para-Toluolsulfonsäure; Carbonsäuren wie Essigsäure oder Polyphosphorsäuren.

Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin; anorganische Basen wie Kaliumphosphat, Kaliumcarbonat und Natriumhydrid.

Ein Beispiel für ein geeignetes Chlorierungsmittel ist Phosphoroxychlorid.

Die Reaktion kann im Vakuum, bei Normaldruck oder unter Überdruck und bei Temperaturen von 0 °C bis 200 °C durchgeführt werden.

### Schritt c)

Verbindungen der Formel (XXIV) können hergestellt werden, beispielsweise in Analogie zu den in Journal of Organic Chemistry 2005, 70, 2696-2700 beschriebenen Verfahren, durch einen Halogen-Sulfon-Austausch ausgehend von Verbindungen der Formel (XXII) mit einer Verbindung der Formel (XXIII) in Gegenwart einer Kupferquelle.

Verbindungen der Formel (XXIII) sind entweder kommerziell erhältlich oder können nach bekannten Methoden hergestellt werden, beispielsweise analog der in Tetrahedron Letters, 2014, 55, 3851-3855 beschriebenen Verfahren.

Die Umsetzung der Verbindung der Formel (XXII) mit der Verbindung der Formel (XXIII) und der Kupferquelle wird generell in einem Lösungsmittel durchgeführt. Bevorzugt werden polar aprotische Lösungsmittel wie beispielsweise Dimethylsulfoxid und N,N-Dimethylformamid eingesetzt.

Als Kupferquelle wird meist CuBr, CuI oder Cu(OAc)₂ verwendet.

Beispiele für geeignete Schwefel-Reagenzien sind Natriumsalze der Sulfinsäuren.

Die Reaktion kann im Vakuum, bei Normaldruck oder unter Überdruck und bei Temperaturen von 20 °C bis 200 °C durchgeführt werden.

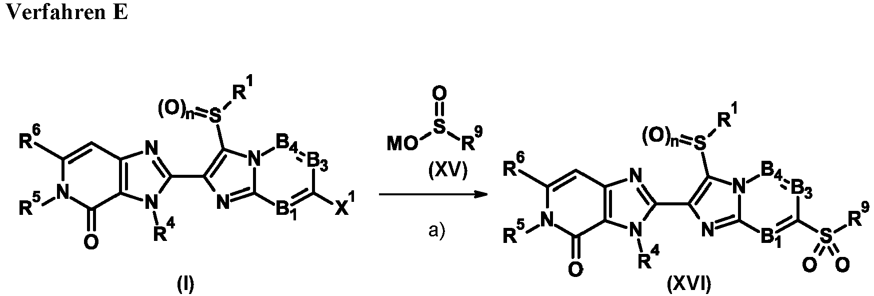

Die Reste R¹, R⁴, R⁵, R⁶, B¹, B³, B⁴, n und R⁹ haben die oben beschriebenen Bedeutungen gemäß Formel (Ia) oder (Ib), X¹ steht für Halogen. M steht für ein Metallatom, insbesondere Li, Na oder K.

### Schritt a)

Verbindungen der Formel (XVI) können hergestellt werden, beispielsweise in Analogie zu den in Journal of Organic Chemistry 2005, 70, 2696-2700 beschriebenen Verfahren, durch einen Halogen-Sulfon-Austausch ausgehend von Verbindungen der Formel (Ia) oder (Ib) mit einer Verbindung der Formel (XV) in Gegenwart einer Kupferquelle.

Verbindungen der Formel (XV) sind entweder kommerziell erhältlich oder können nach bekannten Methoden hergestellt werden, beispielsweise analog der in Tetrahedron Letters, 2014, 55, 3851-3855 beschriebenen Verfahren.

Die Umsetzung der Verbindung der Formel (Ia) oder (Ib) mit der Verbindung der Formel (XV) und der Kupferquelle wird generell in einem Lösungsmittel durchgeführt. Bevorzugt werden polar aprotische Lösungsmittel wie beispielsweise Dimethylsulfoxid und N,N-Dimethylformamid eingesetzt.

Als Kupferquelle wird meist CuBr, CuI oder Cu(OAc)₂ verwendet.

Beispiele für geeignete Schwefel-Reagenzien sind Natriumsalze der Sulfinsäuren.

Die Reaktion kann im Vakuum, bei Normaldruck oder unter Überdruck und bei Temperaturen von 20°C °C bis 200 °C durchgeführt werden.

Die entsprechenden Verbindungen mit n=0 oder n=1 lassen sich analog herstellen.

### Verfahren und Verwendungen

Die Erfindung betrifft auch Verfahren zur Bekämpfung von tierischen Schädlingen, bei dem man Verbindungen der Formel (Ia) oder (Ib) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (Ia) oder (Ib) als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (Ia) oder (Ib) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Vorzugsweise ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt alle Gebiete, technischen Felder und industriellen Anwendungen ab, bei denen diese Hygienemaßnahmen, -vorschriften und -verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden.

Die Verbindungen der Formel (Ia) oder (Ib) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z. B. Geophilus spp., Scutigera spp.;
aus der Ordnung oder der Klasse der Collembola z. B. Onychiurus armatus; Sminthurus viridis;
aus der Klasse der Diplopoda z. B. Blaniulus guttulatus;
aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;
aus der Ordnung der Coleoptera z. B. Acalymma vittatum, Acanthoscelides obtectus, Adoretus spp., Aethina tumida, Agelastica alni, Agrilus spp., z. B. Agrilus planipennis, Agrilus coxalis, Agrilus bilineatus, Agrilus anxius, Agriotes spp., z. B. Agriotes linneatus, Agriotes mancus, Alphitobius diaperinus, Amphimallon solstitialis, Anobium punctatum, Anoplophora spp., z. B. Anoplophora glabripennis, Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dendroctonus spp., z. B. Dendroctonus ponderosae, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnostema consanguinea, Lasioderma serricorne, Latheticus oryzae, Lathridius spp., Lema spp., Leptinotarsa decemlineata, Leucoptera spp., z. B. Leucoptera coffeella, Limonius ectypus, Lissorhoptrus oryzophilus, Listronotus (=Hyperodes) spp., Lixus spp., Luperodes spp., Luperomorpha xanthodera, Lyctus spp., Megacyllene spp., z. B. Megacyllene robiniae, Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Scolytus spp., z. B. Scolytus multistriatus, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;
aus der Ordnung der Dermaptera z. B. Anisolabis maritime, Forficula auricularia, Labidura riparia;
aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;
aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;
aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., z. B. Sirex noctilio, Solenopsis invicta, Tapinoma spp., Technomyrmex albipes, Urocerus spp., Vespa spp., z. B. Vespa crabro, Wasmannia auropunctata, Xeris spp.;
aus der Ordnung der Isopoda z. B. Armadillidium vulgare, Oniscus asellus, Porcellio scaber;
aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;
aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Dioryctria spp., z. B. Dioryctria zimmermani, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens , Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Podesia spp., z. B. Podesia syringae, Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;
aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;
aus der Ordnung der Phthiraptera z. B. Damalinia spp., Haematopinus spp., Linognathus spp., Pediculus spp., Phylloxera vastatrix, Phthirus pubis, Trichodectes spp.;
aus der Ordnung der Psocoptera z. B. Lepinotus spp., Liposcelis spp.;
aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;
aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z. B. Scutigerella spp., z. B. Scutigerella immaculata;
Schädlinge aus dem Stamm der Mollusca, z. B. aus der Klasse der Bivalvia, z. B. Dreissena spp.;
sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;
Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus, Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella ornata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

Die Verbindungen der Formel (Ia) oder (Ib) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (Ia) oder (Ib). Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanol-alkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einer oder mehreren Verbindungen der Formel (Ia) oder (Ib) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (Ia) oder (Ib) mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (Ia) oder (Ib) oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), der Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid), der Carbonate und der Nitrile.

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid oder Dimethylsulfoxid, Carbonate wie Propylencarbonat, Butylencarbonat, Diethylcarbonat oder Dibutylcarbonat, oder Nitrile wie Acetonitril oder Propannitril.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid, Carbonate wie Propylencarbonat, Butylencarbonat, Diethylcarbonat oder Dibutylcarbonat, Nitrile wie Acetonitril oder Propannitril, sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Isethionatderivate, Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (Ia) oder (Ib) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (Ia) oder (Ib) mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (Ia) oder (Ib), besonders bevorzugt zwischen 0,01 und 95 Gew.-% der Verbindung der Formel (Ia) oder (Ib), ganz besonders bevorzugt zwischen 0,5 und 90 Gew.-% der Verbindung der Formel (Ia) oder (Ib), bezogen auf das Gewicht der Formulierung.

Der Gehalt an der Verbindung der Formel (Ia) oder (Ib) in den aus den Formulierungen bereiteten Anwendungsformen (insbesondere Schädlingsbekämpfungsmittel) kann in weiten Bereichen variieren. Die Konzentration der Verbindung der Formel (Ia) oder (Ib) in den Anwendungsformen kann üblicherweise zwischen 0,00000001 und 95 Gew.-% der Verbindung der Formel (Ia) oder (Ib), vorzugsweise zwischen 0,00001 und 1 Gew.-%, bezogen auf das Gewicht der Anwendungsform, liegen. Die Anwendung geschieht in einer den Anwendungsformen angepassten üblichen Weise.

### Mischungen

Die Verbindungen der Formel (Ia) oder (Ib) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (Ia) oder (Ib) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (Ia) oder (Ib) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (Ia) oder (Ib) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

### Insektizide/Akarizide/Nematizide

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.
(1) Acetylcholinesterase(AChE)-Inhibitoren, vorzugsweise Carbamate ausgewählt aus Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb, oder Organophosphate ausgewählt aus Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Blocker, vorzugsweise Cyclodien-organochlorine ausgewählt aus Chlordan und Endosulfan, oder Phenylpyrazole (Fiprole) ausgewählt aus Ethiprol und Fipronil.
(3) Natrium-Kanal-Modulatoren, vorzugsweise Pyrethroide ausgewählt aus Acrinathrin, Allethrin, d-cistrans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin, oder DDT oder Methoxychlor.
(4) Kompetitive Modulatoren des nicotinischen Acetylcholin-Rezeptors (nAChR), vorzugsweise Neonicotinoide ausgewählt aus Acetamiprid, Clothianidin, Dinotefuran, Imidacloprid, Nitenpyram, Thiacloprid und Thiamethoxam, oder Nicotin, oder Sufoximine ausgewählt aus Sulfoxaflor, oder Butenolide ausgewählt aus Flupyradifurone.
(5) Allosterische Modulatoren des nicotinischen Acetylcholin-Rezeptors (nAChR), vorzugsweise Spinosyne ausgewählt aus Spinetoram und Spinosad.
(6) Allosterische Modulatoren des Glutamat-abhängigen Chloridkanals (GluCl), vorzugsweise Avermectine/Milbemycine ausgewählt aus Abamectin, Emamectin-benzoat, Lepimectin und Milbemectin.
(7) Juvenilhormon-Mimetika, vorzugsweise Juvenilhormon-Analoge ausgewählt aus Hydropren, Kinopren und Methopren, oder Fenoxycarb oder Pyriproxyfen.
(8) Verschiedene nicht spezifische (multi-site) Inhibitoren, vorzugsweise Alkylhalogenide ausgewählt aus Methylbromid und andere Alkylhalogenide, oder Chloropicrin oder Sulfurylfluorid oder Borax oder Brechweinstein oder Methylisocyanaterzeuger ausgewählt aus Diazomet und Metam.
(9) TRPV-Kanal-Modulatoren chordotonaler Organe ausgewählt aus Pymetrozin und Pyrifluquinazon.
(10) Milbenwachstumsinhibitoren ausgewählt aus Clofentezin, Hexythiazox, Diflovidazin und Etoxazol.
(11) Mikrobielle Disruptoren der Insektendarmmembran ausgewählt aus *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und *B.t.-*Pflanzenproteine ausgewählt aus Cry1Ab, Cry1Ac, Cry1Fa, Cry1A.105, Cry2Ab, VIP3A, mCry3A, Cry3Ab, Cry3Bb und Cry34Abl/35Abl.
(12) Inhibitoren der mitochondrialen ATP-Synthase, vorzugsweise ATP-Disruptoren ausgewählt aus Diafenthiuron, oder Organozinnverbindungen ausgewählt aus Azocyclotin, Cyhexatin und Fenbutatinoxid, oder Propargit oder Tetradifon.
(13) Entkoppler der oxidativen Phoshorylierung durch Störung des Protonengradienten ausgewählt aus Chlorfenapyr, DNOC und Sulfluramid.
(14) Blocker des nicotinischen Acetylcholinrezeptorkanals ausgewählt aus Bensultap, Cartaphydrochlorid, Thiocyclam und Thiosultap-Natrium.
(15) Inhibitoren der Chitinbiosynthese, Typ 0, ausgewählt aus Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1 ausgewählt aus Buprofezin.
(17) Häutungsdisruptor (insbesondere bei Dipteren, d. h. Zweiflüglern) ausgewählt aus Cyromazin.
(18) Ecdyson-Rezeptor-Agonisten ausgewählt aus Chromafenozid, Halofenozid, Methoxyfenozid und Tebufenozid.
(19) Oktopamin-Rezeptor-Agonisten ausgewählt aus Amitraz.
(20) Mitochondriale Komplex-III-Elektronentransportinhibitoren ausgewählt aus Hydramethylnon, Acequinocyl und Fluacrypyrim.
(21) Mitochondriale Komplex-I-Elektronentransportinhibitoren, vorzugsweise METI-Akarizide ausgewählt aus Fenazaquin, Fenpyroximat, Pyrimidifen, Pyridaben, Tebufenpyrad und Tolfenpyrad, oder Rotenon (Derris).
(22) Blocker des spannungsabhängigen Natriumkanals ausgewählt aus Indoxacarb und Metaflumizone.
(23) Inhibitoren der Acetyl-CoA-Carboxylase, vorzugsweise Tetron- und Tetramsäurederivate ausgewählt aus Spirodiclofen, Spiromesifen und Spirotetramat.
(24) Inhibitoren des mitochondrialen Komplex-IV-Elektronentransports, vorzugsweise Phosphine ausgewählt aus Aluminiumphosphid, Calciumphosphid, Phosphin und Zinkphosphid, oder Cyanide ausgewählt aus Calciumcyanid, Kaliumcyanid und Natriumcyanid.
(25) Inhibitoren des mitochondrialen Komplex-II-Elektronentransports, vorzugsweise beta-Ketonitrilderivate ausgewählt aus Cyenopyrafen und Cyflumetofen, oder Carboxanilide ausgewählt aus Pyflubumid.
(28) Ryanodinrezeptor-Modulatoren, vorzugsweise Diamide ausgewählt aus Chlorantraniliprol, Cyantraniliprol und Flubendiamid.
(29) Modulatoren chordotonaler Organe (mit undefinierter Zielstruktur) ausgewählt aus Flonicamid.
(30) weitere Wirkstoffe ausgewählt aus Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tigolaner, Tioxazafen, Thiofluoximat, Triflumezopyrim und Iodmethan; des Weiteren Präparate auf Basis von *Bacillus firmus* (1-1582, BioNeem, Votivo), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl] -5 -fluorspiro [indol-3,4'-piperidin] -1(2H)-yl} (2-chlorpyridin-4-yl)methanon (bekannt aus WO2003/106457) (CAS 637360-23-7), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl]-2-(3-chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-1-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfmyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e][1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluonnethylphenoxy)-3-(6-trifluonnethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8), N-[3-Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9) und N-[4-(Aminothioxomethyl)-2-methyl-6-[(methylamino)carbonyl]phenyl]-3-bromo-l-(3-chloro-2-pyridinyl)- 1H-pyrazol-5-carboxamid (bekannt aus CN 103265527 A) (CAS 1452877-50-7), 5-(1,3-Dioxan-2-yl)-4-[[4-(trifluormethyl)phenyl]methoxy]-pyrimidin (bekannt aus WO 2013/115391 A1) (CAS 1449021-97-9), 3-(4-Chlor-2,6-dimethylphenyl)-4-hydroxy-8-methoxy-1-methyl-1,8-diazaspiro[4.5]dec-3-en-2-on (bekannt aus WO 2010/066780 A1, WO 2011/151146 A1) (CAS 1229023-34-0), 3-(4-Chlor-2,6-dimethylphenyl)-8-methoxy-1-methyl-1,8-diazaspiro[4.5]decane-2,4-dion (bekannt aus WO 2014/187846 A1) (CAS 1638765-58-8), 3-(4-Chlor-2,6-dimethylphenyl)-8-methoxy-1-methyl-2-oxo-1, 8-diazaspiro[4.5]dec-3-en-4-yl-carbonsäureethylester (bekannt aus WO 2010/066780 A1, WO 2011151146 A1) (CAS 1229023-00-0), N-[1-[(6-chlor-3-pyridinyl)methyl]-2(1H)-pyridinylidene]-2,2,2-trifluor-acetamide (bekannt aus DE 3639877 A1, WO 2012029672 A1) (CAS 1363400-41-2), [N(*E*)]-N-[1-[(6-chlor-3-pyridinyl)methyl]-2(1*H*)-pyridinylidene]-2,2,2-trifluor-acetamide (bekannt aus WO 2016005276 A1) (CAS 1689566-03-7), [N(*Z*)]-N-[1-[(6-chlor-3-pyridinyl)methyl]-2(1*H*)-pyridinylidene]-2,2,2-trifluor- acetamide (CAS 1702305-40-5), 3-*endo*-3-[2-Propoxy-4-(trifluormethyl) phenoxy]-9-[[5-(trifluormethyl)-2-pyridinyl]oxy]-9-azabicyclo[3.3.1]nonan (bekannt aus WO 2011/105506 A1, WO 2016/133011 A1) (CAS 1332838-17-1).

### Fungizide

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.
1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-1-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl]-1-(1H-1,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3 -(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-yl-thiocyanat, (1.040) 1-{[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,SR)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2-[4-(4-Chlorphenoxy)-2-(trifluormethyl)phenyl]-1-(1H-1,2,4-triazol-1-yl)pentan-2-ol, (1.055) 2-[4-(4-Chlorphenoxy)-2-(trifluormethyl)phenyl]-1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1-{[3 -(2-chlorphenyl)-2-(2,4-difluorphenyl)oxiran-2-yl]methyl}-1H-1,2,4-triazol, (1.061) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4-{[3-(2,2,3,3-tetrafluorpropoxy)phenyl]sulfanyl}phenyl)-N-ethyl-N-methylimidoformamid, (1.066) N'-(2,5-Dimethyl-4-{[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068) N'-(2,5-Dimethyl-4-{3-[(2,2,2-trifluorethyl)sulfanyl]phenoxy}phenyl)-N-ethyl-N-methylimidoformamid, (1.069) N'-(2,5-Dimethyl-4-{3-[(2,2,3,3-tetrafluorpropyl)sulfanyl]phenoxy}phenyl)-N-ethyl-N-methylimidoformamid, (1.070) N'-(2,5-Dimethyl-4-{3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4-{[3-(Difluormethoxy)phenyl]sulfanyl}-2,5-dimethylphenyl)-N-ethyl-N-methylimidoformamid, (1.073) N'-(4-{3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-{4-[(4,5-Dichlor-1,3-thiazol-2-yl)oxy]-2,5-dimethylphenyl}-N-ethyl-N-methylimidoformamid, (1.076) N'-{5-Brom-6-[(1R)-1-(3,5-difluorophenyl)ethoxy]-2-methylpyridin-3-yl}-N-ethyl-N-methylimidoformamid, (1.077) N'-{5-Brom-6-[(1S)-1-(3,5-difluorphenyl)ethoxy]-2-methylpyridin-3-yl}-N-ethyl-N-methylimidoformamid, (1.078) N'-{5-Brom-6-[(cis-4-isopropylcyclohexyl)oxy]-2-methylpyridin-3-yl}-N-ethyl-N-methylimidoformamid, (1.079) N'-{5-Brom-6-[(trans-4-isopropylcyclohexyl)oxy]-2-methylpyridin-3-yl}-N-ethyl-N-methylimidoformamid, (1.080) N'-{5-Bromo-6-[1-(3,5-difluorphenyl)ethoxy]-2-methylpyridin-3-yl}-N-ethyl-N-methylimidoformamid, (1.081) Mefentrifluconazole, (1.082) Ipfentrifluconazole.
2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des anti-epimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl- 1H-pyrazol-4-carboxamid, (2.033) 5,8-Difluor-N-[2-(2-fluor-4- {[4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1H-pyrazol-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-[1-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.
3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{[1-(4-Chlorphenyl)-1H-pyrazol-3-yl]oxy}-2-(methoxyimino)-N,3-dimethylpent-3-enamid, (3.023) (2R)-2-{2-[(2,5-Dimethylphenoxy)methyl]phenyl}-2-methoxy-N-methylacetamid, (3.024) (2S)-2-{2-[(2,5-Dimethylphenoxy)methyl]phenyl}-2-methoxy-N-methylacetamid, (3.025) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl-2-methylpropanoat, (3.026) 2-{2-[(2,5-Dimethylphenoxy)methyl]phenyl} -2-methoxy-N-methylacetamid, (3.027) N-(3-Ethyl-3,5,5-trimethylcyclohexyl)-3-formamido-2-hydroxybenzamid, (3.028) (2E,3Z)-5-{[1-(4-Chlor-2-fluorphenyl)-1H-pyrazol-3-yl]oxy}-2-(methoxyimino)-N,3-dimethylpent-3-enamid, (3.029) Methyl {5-[3-(2,4-dimethylphenyl)-1H-pyrazol-1-yl] -2-methylbenzyl}carbamate.
4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.
5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram, (5.023) 6-Ethyl-5,7-dioxo-6,7-dihydro-5H-pyrrolo[3',4':5,6][1,4]dithiino[2,3-c][1,2]thiazole-3-carbonitrile.
6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.
7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.
(8) Inhibitoren der ATP-Produktion, beispielsweise (8.001) Silthiofam.
9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.
10) Inhibitoren der Lipid- und Membran-Synthese, beispielsweise (10.001) Propamocarb, (10.002) Propamocarbhydrochlorid, (10.003) Tolclofos-Methyl.
11) Inhibitoren der Melanin-Biosynthese, beispielsweise (11.001) Tricyclazol, (11.002) 2,2,2-Trifluorethyl-{3-methyl-1-[(4-methylbenzoyl)amino]butan-2-yl}carbamat.
12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).
13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.
14) Verbindungen, die als Entkoppler wirken können, beispielsweise (14.001) Fluazinam, (14.002) Meptyldinocap.
15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarb-fosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2-{2-Fluor-6-[(8-fluor-2-methylquinolin-3-yl)oxy]phenyl}propan-2-ol, (15.043) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1-yl)quinolin, (15.047) 3-(4,4-Difluor-3,3-dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5 -(quinolin-3 -yl)-2,3 -dihydro-1,4-benzoxazepin, (15.055) But-3-yn-1-yl {6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl {6-[({[(1-methyl-1H-tetrazol-5-yl)(phenyl)methylene]amino}oxy)methyl]pyridin-2-yl}carbamat, (15.062) 5-Fluor-4-imino-3-methyl-1-[(4-methylphenyl)sulfonyl]-3,4-dihydropyrimidin-2(1H)-one.

### Biologische Schädlingsbekämpfungsmittel als Mischungskomponenten

Die Verbindungen der Formel (Ia) oder (Ib) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens*, Stamm FZB42 (DSM 231179), oder *Bacillus cereus*, insbesondere *B. cereus* Stamm CNCM I-1562 oder *Bacillus firmus*, Stamm I-1582 (Accession number CNCM 1-1582) oder *Bacillus pumilus*, insbesondere Stamm GB34 (Accession No. ATCC 700814) und Stamm QST2808 (Accession No. NRRL B-30087), oder *Bacillus subtilis*, insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis*, insbesondere *B. thuringiensis* Subspezies *israelensis* (Serotyp H-14), Stamm AM65-52 (Accession No. ATCC 1276), oder *B. thuringiensis subsp. aizawai*, insbesondere Stamm ABTS-1857 (SD-1372), oder *B*. *thuringiensis subsp. kurstaki* Stamm HD-1, oder *B. thuringiensis subsp. tenebrionis* Stamm NB 176 (SD-5428), *Pasteuria penetrans*, *Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana*, insbesondere Stamm ATCC 74040, *Coniothyrium minitans*, insbesondere Stamm CON/M/91-8 (Accession No. DSM-9660), *Lecanicillium spp.*, insbesondere Stamm HRO LEC 12, *Lecanicillium lecanii* (ehemals bekannt als *Verticillium lecanii*), insbesondere Stamm KV01, *Metarhizium anisopliae*, insbesondere Stamm F52 (DSM3884/ ATCC 90448), *Metschnikowia fructicola*, insbesondere Stamm NRRL Y-30752, *Paecilomyces fumosoroseus (*neu: *Isaria fumosorosea)*, insbesondere Stamm IFPC 200613, oder Stamm Apopka 97 (Accesion No. ATCC 20874), *Paecilomyces lilacinus*, insbesondere *P. lilacinus* Stamm 251 (AGAL 89/030550), *Talaromyces flavus*, insbesondere Stamm V117b, *Trichoderma atroviride*, insbesondere Stamm SC1 (Accession Number CBS 122089), *Trichoderma harzianum*, insbesondere *T. harzianum rifai T39.* (Accession Number CNCM 1-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp.*, *Burkholderia spp.*, insbesondere *Burkholderia cepacia* (ehemals bekannt als *Pseudomonas cepacia*), *Gigaspora spp.*, oder *Gigaspora monosporum*, *Glomus spp.*, *Laccaria spp.*, *Lactobacillus buchneri*, *Paraglomus spp.*, *Pisolithus tinctorus*, *Pseudomonas spp.*, *Rhizobium spp.*, insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem ^{™} Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischungskomponenten

Die Verbindungen der Formel (Ia) oder (Ib) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (Ia) oder (Ib) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (Ia) oder (Ib) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (Ia) oder (Ib) nach dem Ultra-Low-Volume-Verfahren auszubringen oder die Anwendungsform oder die Verbindung der Formel (Ia) oder (Ib) selbst in den Boden zu injizieren.

Eine bevorzugte direkte Behandlung der Pflanzen ist die Blattapplikation, d. h. die Verbindungen der Formel (Ia) oder (Ib) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (Ia) oder (Ib) auch über das Wurzelwerk in die Pflanzen. Die Behandlung der Pflanzen erfolgt dann durch Einwirkung der Verbindungen der Formel (Ia) oder (Ib) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (Ia) oder (Ib) getränkt, oder durch die Bodenapplikation, d. h. die erfindungsgemäßen Verbindungen der Formel (Ia) oder (Ib) werden in fester Form (z. B. in Form eines Granulats) in den Standort der Pflanzen eingebracht, oder durch Tropfapplikation ("drip", oftmals auch als "Chemigation" bezeichnet), d.h. die erfindungsgemäßen Verbindungen der Formel (Ia) oder (Ib) werden mittels Oberflächen- oder Untergrund-Tropfrohren über bestimmte Zeiträume zusammen mit variierenden Mengen an Wasser an definierten Stellen in der Nähe der Pflanzen eingebracht. Bei Wasserreiskulturen kann das auch durch Zudosieren der Verbindung der Formel (Ia) oder (Ib) in einer festen Anwendungsform (z. B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Be-handlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (Ia) oder (Ib) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (Ia) oder (Ib) und einer Mischungskomponente behandelt wird. Es umfasst ferner auch ein Verfahren, in dem das Saatgut zu unterschiedlichen Zeiten mit einer Verbindung der Formel (Ia) oder (Ib) und einer Mischungskomponente behandelt wird.

Die Erfindung bezieht sich ebenfalls auf die Verwendung der Verbindungen der Formel (Ia) oder (Ib) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (Ia) oder (Ib) behandelt wurde. Die Erfindung bezieht sich auch auf Saatgut, welches zur gleichen Zeit mit einer Verbindung der Formel (Ia) oder (Ib) und einer Mischungskomponente behandelt wurde. Die Erfindung bezieht sich weiterhin auf Saatgut, welches zu unterschiedlichen Zeiten mit einer Verbindung der Formel (Ia) oder (Ib) und einer Mischungskomponente behandelt wurde. Bei Saatgut, welches zu unterschiedlichen Zeiten mit einer Verbindung der Formel (Ia) oder (Ib) und einer Mischungskomponente behandelt wurde, können die einzelnen Substanzen in unterschiedlichen Schichten auf dem Saatgut vorhanden sein. Dabei können die Schichten, die eine Verbindung der Formel (Ia) oder (Ib) und Mischungskomponenten enthalten, gegebenenfalls durch eine Zwischenschicht getrennt sein. Die Erfindung bezieht sich auch auf Saatgut, bei dem eine Verbindung der Formel (Ia) oder (Ib) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (Ia) oder (Ib) einem Filmcoating-Verfahren unterzogen wird, um Staubabrieb am Saatgut zu vermeiden.

Einer der auftretenden Vorteile, wenn eine Verbindung der Formel (Ia) oder (Ib) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (Ia) oder (Ib) Keimung und Auflauf des behandelten Saatguts gefördert werden können.

Ebenso ist es als vorteilhaft anzusehen, dass Verbindungen der Formel (Ia) oder (Ib) insbesondere auch bei transgenem Saatgut eingesetzt werden können.

Verbindungen der Formel (Ia) oder (Ib) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (Ia) oder (Ib) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (Ia) oder (Ib) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (Ia) oder (Ib) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (Ia) oder (Ib) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (Ia) oder (Ib) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (Ia) oder (Ib) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (Ia) oder (Ib) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formu-lierungen enthalten sein können, kommen alle für derartige Zwecke in agrochemischen Mitteln ein-setzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (Ia) oder (Ib) in den Formulierungen und nach dem Saatgut. Die Aufwandmengen bei der Verbindung der Formel (Ia) oder (Ib) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d. h. dem Gebiet der Tiermedizin, sind die Verbindungen der Formel (Ia) oder (Ib) gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (Ia) oder (Ib), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische .

Gemäß einer bestimmten Ausführungsform werden die Verbindungen der Formel (Ia) oder (Ib) an Säugetiere verabreicht.

Gemäß einer weiteren bestimmten Ausführungsform werden die Verbindungen der Formel (Ia) oder (Ib) an Vögel, nämlich Stubenvögel oder insbesondere Geflügel, verabreicht.

Durch Verwendung der Verbindungen der Formel (Ia) oder (Ib) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (Ia) oder (Ib) wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (Ia) oder (Ib) den jeweiligen Parasiten abtöten, sein Wachstum verhindern oder seine Vermehrung verhindern.

Zu den Arthropoden zählen beispielsweise, ohne hierauf beschränkt zu sein,
aus der Ordnung Anoplurida zum Beispiel Haematopinus spp., Linognathus spp., Pediculus spp., Phtirus spp., Solenopotes spp.;
aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;
aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;
aus der Ordnung Siphonapterida, zum Beispiel Ceratophyllus spp., Ctenocephalides spp., Pulex spp., Tunga spp., Xenopsylla spp.;
aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Stemostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:
Metamonada: aus der Ordnung Diplomonadida zum Beispiel Giardia spp., Spironucleus spp. Parabasala: aus der Ordnung Trichomonadida zum Beispiel Histomonas spp., Pentatrichomonas spp., Tetratrichomonas spp., Trichomonas spp., Tritrichomonas spp.

Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z.B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:
Monogenea: z. B.: Dactylogyrus spp., Gyrodactylus spp., Microbothrium spp., Polystoma spp., Troglecephalus spp.;
Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.

Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.

Nematoden: aus der Ordnung Trichinellida zum Beispiel: Capillaria spp., Trichinella spp., Trichomosoides spp., Trichuris spp.

Aus der Ordnung Tylenchida zum Beispiel: Micronema spp., Parastrangyloides spp., Strongyloides spp.

Aus der Ordnung Rhabditina zum Beispiel: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.

Aus der Ordnung Spirurida zum Beispiel: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acanthocephala: aus der Ordnung Oligacanthorhynchida z.B: Macracanthorhynchus spp., Prosthenorchis spp.; aus der Ordnung Moniliformida zum Beispiel: Moniliformis spp.,

Aus der Ordnung Polymorphida zum Beispiel: Filicollis spp.; aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der Verbindungen der Formel (Ia) oder (Ib) nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen.

So bezieht sich eine Ausführungsform der vorliegenden Erfindung auf die Verbindungen der Formel (Ia) oder (Ib) zur Verwendung als Arzneimittel.

Ein weiterer Aspekt bezieht sich auf die Verbindungen der Formel (Ia) oder (Ib) zur Verwendung als Antiendoparasitikum.

Ein weiterer spezieller Aspekt der Erfindung betrifft die Verbindungen der Formel (Ia) oder (Ib) zur Verwendung als Antihelminthikum, insbesondere zur Verwendung als Nematizid, Platymelminthizid, Acanthocephalizid oder Pentastomizid.

Ein weiterer spezieller Aspekt der Erfindung betrifft die Verbindungen der Formel (Ia) oder (Ib) zur Verwendung als Antiprotozoikum.

Ein weiterer Aspekt betrifft die Verbindungen der Formel (Ia) oder (Ib) zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (Ia) oder (Ib) und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (Ia) oder (Ib) mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder herkömmlicherweise in veterinärmedizinischen Formulierungen verwendeten Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe nematizider, platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (Ia) oder (Ib) bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (Ia) oder (Ib) bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (Ia) oder (Ib) mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alanwood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akkarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren;
Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;
Verbindungen aus anderen Klassen, z.B. Butacarb, Dimetilan, Cloethocarb, Phosphocarb, Pirimiphos(-ethyl), Parathion(-ethyl), Methacrifos, Isopropyl-o-salicylat, Trichlorfon, Tigolaner, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;
Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;
Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),
Neonicotinoide, z. B. Nithiazin
Dicloromezotiaz, Triflumezopyrim makrocyclische Lactone, z. B. Nemadectin, Ivermectin, Latidectin, Moxidectin, Selamectin, Eprinomectin, Doramectin, Emamectinbenzoat; Milbemycinoxim
Tripren, Epofenonan, Diofenolan;
Biologicals, Hormone oder Pheromone, zum Beispiel natürliche Produkte, z.B. Thuringiensin, Codlemon oder Neem-Komponenten
Dinitrophenole, z. B. Dinocap, Dinobuton, Binapacryl;
Benzoylharnstoffe, z. B. Fluazuron, Penfluron,
Amidinderivate, z. B. Chlormebuform, Cymiazol, Demiditraz
Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.

Zu den anthelmintischen Wirkstoffen zählen, ohne hierauf beschränkt zu sein, die folgenden nematiziden, trematiziden und/oder cestoziden Wirkstoffe:
aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;
aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;
aus der Klasse der Depsipeptide, vorzugsweise cyclischen Depsipetide, insbesondere 24-gliedrigen cyclischen Depsipeptide, zum Beispiel: Emodepsid, PF1022A;
aus der Klasse der Tetrahydropyrimidine zum Beispiel: Morantel, Pyrantel, Oxantel;
aus der Klasse der Imidazothiazole zum Beispiel: Butamisol, Levamisol, Tetramisol;
aus der Klasse der Aminophenylamidine zum Beispiel: Amidantel, deacyliertes Amidantel (dAMD), Tribendimidin;
aus der Klasse der Aminoacetonitrile zum Beispiel: Monepantel;
aus der Klasse der Paraherquamide zum Beispiel: Paraherquamid, Derquantel;
aus der Klasse der Salicylanilide zum Beispiel: Tribromsalan, Bromoxanid, Brotianid, Clioxanid, Closantel, Niclosamid, Oxyclozanid, Rafoxanid;
aus der Klasse der substituierten Phenole zum Beispiel: Nitroxynil, Bithionol, Disophenol, Hexachlorophen, Niclofolan, Meniclopholan;
aus der Klasse der Organophosphate zum Beispiel: Trichlorfon, Naphthalofos, Dichlorvos/DDVP, Crufomat, Coumaphos, Haloxon;
aus der Klasse der Piperazinone/Chinoline zum Beispiel: Praziquantel, Epsiprantel;
aus der Klasse der Piperazine zum Beispiel: Piperazin, Hydroxyzin;
aus der Klasse der Tetracycline zum Beispiel: Tetracyclin, Chlorotetracyclin, Doxycyclin, Oxytetracyclin, Rolitetracyclin;
aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:
aus der Klasse der Triazine zum Beispiel: Diclazuril, Ponazuril, Letrazuril, Toltrazuril;
aus der Klasse Polyletherionophor zum Beispiel: Monensin, Salinomycin, Maduramicin, Narasin;
aus der Klasse der makrocyclischen Lactone zum Beispiel: Milbemycin, Erythromycin;
aus der Klasse der Chinolone zum Beispiel: Enrofloxacin, Pradofloxacin;
aus der Klasse der Chinine zum Beispiel: Chloroquin;
aus der Klasse der Pyrimidine zum Beispiel: Pyrimethamin;
aus der Klasse der Sulfonamide zum Beispiel: Sulfachinoxalin, Trimethoprim, Sulfaclozin;
aus der Klasse der Thiamine zum Beispiel: Amprolium;
aus der Klasse der Lincosamide zum Beispiel: Clindamycin;
aus der Klasse der Carbanilide zum Beispiel: Imidocarb;
aus der Klasse der Nitrofurane zum Beispiel: Nifurtimox;
aus der Klasse der Chinazolinonalkaloide zum Beispiel: Halofuginon;
aus diversen anderen Klassen zum Beispiel: Oxamniquin, Paromomycin;
aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

### Vektorbekämpfung

Die Verbindungen der Formel (Ia) oder (Ib) können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von anderen Würmern;
   - Aedes: Gelbfieber, Dengue-Fieber, weitere virale Erkrankungen, Filariasis;
   - Simulien: Übertragung von Würmern, insbesondere Onchocerca volvulus;
   - Psychodidae: Übertragung von Leishmaniose
2) Läuse: Hautinfektionen, epidemisches Fleckfieber;
3) Flöhe: Pest, endemisches Fleckfieber, Bandwürmer;
4) Fliegen: Schlafkrankheit (Trypanosomiasis); Cholera, weitere bakterielle Erkrankungen;
5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;
6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (Ia) oder (Ib) Resistenzbrechend sind.

Verbindungen der Formel (Ia) oder (Ib) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (Ia) oder (Ib) zur Vektorbekämpfung, z. B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (Ia) oder (Ib) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (Ia) oder (Ib) zusammen mit mindestens einem weiteren Insektizid und/oder mindestens einem Fungizid eingesetzt.

In einer weiteren Ausführungsform liegen die Verbindungen der Formel (Ia) oder (Ib) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (Ia) oder (Ib) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (Ia) oder (Ib) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (Ia) oder (Ib) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (Ia) oder (Ib) allein oder in Kombination mit anderen Wirk- und/oder Hilfsstoffen verwendet. Bevorzugt werden sie in Haushaltsinsektizid-Produkten verwendet. Die Verbindungen der Formel (Ia) oder (Ib) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

**2-[3-(Ethylsulfonyl)-7-(trifluormethyl)imidazo[1,2-a]pyridin-2-yl]-3,5-dimethyl-6-(trifluormethyl)-3,5-dihydro-4H-imidazo[4,5-c]pyridin-4-on (I-01)** 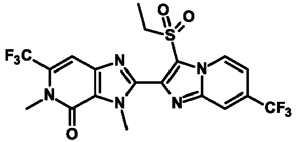 100 mg (0,31 mmol) 3-(Ethylsulfonyl)-7-(trifluormethyl)imidazo[1,2-a]pyridin-2-carbonsäure, 130 mg (0,34 mmol) HATU und 120 mg (0,93 mmol) Diisoproylethylamin wurde in Tetrahydrofuran (1 mL) vorgelegt und 15 min bei Raumtemperatur gerrührt. Anschließend wurde 75,5 mg (0,34 mmol) 4-Amino-1-methyl-3-(methylamino)-6-(trifluormethyl)pyridin-2(1H)-on zugegeben und das Reaktionsgemisch 16 h bei Raumtemperatur gerührt. Das Reaktionsgemisch wurde unter vermindertem Druck vom Lösungsmittel befreit. Der Rückstand wurde in Essigsäure (15 mL) vorgelegt und bei 90 °C für 12 h gerührt. Der Eisessig wurde im Vakuum entfernt und Phosphorylchlorid (5 mL) zugegeben. Das Gemisch wurde 2 h bei 120 °C gerührt. Nach dem Abkühlen wurde das Gemisch vorsichtig auf Wasser gegeben und die wässrige Phase mit Dichlormethan extrahiert. Die organische Phase wurde über Natriumsulfat getrocknet, filtriert und unter vermindertem Druck vom Lösungsmittel befreit. Das Rohprodukt wurde durch säulenchromatographische Aufreinigung über präparative HPLC gereinigt.

logP (sauer): 3,2; MH⁺: 508; ¹H-NMR(400 MHz, D₆-DMSO) δ ppm: 9.21 (d, 1H), 8.54 (s, 1H), 7.65 (dd, 1H), 7.44 (s, 1H), 4.13 (s, 3H), 3.87 (q, 2H), 3.63 (s, 3H), 1.27 (t, 3H).

**1-{2-[3,5-Dimethyl-4-oxo-6-(trifluormethyl)-4,5-dihydro-3H-imidazo[4,5-c]pyridin-2-yl]-3-(ethylsulfonyl)imidazo[1,2-a]pyridin-7-yl}cyclopropancarbonitril (I-02)** 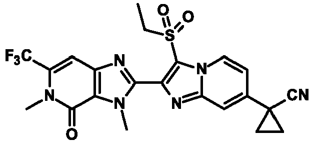 200 mg (0,62 mmol) 7-(1-Cyancyclopropyl)-3-(ethylsulfonyl)imidazo[1,2-a]pyridin-2-carbonsäure, 262 mg (0,68 mmol) HATU und 242 mg (1,87 mmol) Diisoproylethylamin wurde in Tetrahydrofuran (10 mL) vorgelegt und 15 min bei Raumtemperatur gerrührt. Anschließend wurde 152 mg (0,68 mmol) 4-Amino-1-methyl-3-(methylamino)-6-(trifluormethyl)pyridin-2(1H)-on zugegeben und das Reaktionsgemisch 3 Tage bei Raumtemperatur gerührt. Das Reaktionsgemisch wurde unter vermindertem Druck vom Lösungsmittel befreit. Der Rückstand wurde in Dichlormethan aufgenommen und die organische Phase mit 1N HCl (2x) und gesättigter Natriumchloridlösung gewaschen. Die organische Phase wurde über Natriumsulfat getrocknet, filtriert und unter vermindertem Druck vom Lösungsmittel befreit. Der Rückstand wurde in Essigsäure (15 mL) vorgelegt und bei 90 °C für 16 h gerührt. Der Eisessig wurde im Vakuum entfernt und Phosphorylchlorid (10 mL) zugegeben. Das Gemisch wurde 2 h bei 120 °C gerührt. Nach dem Abkühlen wurde das Gemisch vorsichtig auf Wasser gegeben und die wässrige Phase mit Dichlormethan extrahiert. Die organische Phase wurde über Natriumsulfat getrocknet, filtriert und unter vermindertem Druck vom Lösungsmittel befreit. Das Rohprodukt wurde durch säulenchromatographische Aufreinigung über MPLC und präparative HPLC gereinigt.

logP (sauer): 2,59; MH⁺: 505; ¹H-NMR(400 MHz, D₆-DMSO) δ ppm: 9.01 (d, 1H), 7.86 (s, 1H), 7.43 (s, 1H), 7.35 (dd, 1H), 4.12 (s, 3H), 3.82 (q, 2H), 3.63 (s, 3H), 1.96-1.92 (m, 2H), 1.81-1.77 (m, 2H), 1.25 (t, 3H).

**7-(1-Cyancyclopropyl)-3-(ethylsulfonyl)imidazo[1,2-a]pyridin-2-carbonsäure**

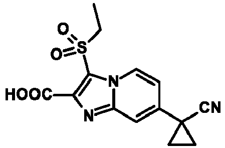

Eine Lösung von Ethyl-7-(1-cyancyclopropyl)-3-(ethylsulfonyl)imidazo[1,2-a]pyridin-2-carboxylat (15 g, Rohprodukt) in Tetrahydrofuran (50 mL) und Wasser (50 mL) wurde auf 0 °C abgekühlt und Lithiumhydroxid Monohydrat (5.44 g, 129.7 mmol) zugegeben. Das Gemisch wurde 15 min bei 0 °C gerührt und anschließen Tetrahydrofuran im Vakuum entfernt. Die verbleibende Lösung wurde mit IN HCl angesäuert (pH 4-5) und mit einem Gemisch aus 10% Methanol in Dichlormethan extrahiert (3x). Die vereinte organische Phase wurde über Natriumsulfat getrocknet und im Vakuum einkonzentriert. Der Rückstand wurde mit Diethylether verrührt und der entstandene Feststoff abfiltriert.

MH⁺: 320; ¹H-NMR (400 MHz, D₆-DMSO) δ ppm: 13.83 (br s, 1H), 8.96 (d, 1H), 7.76 (d, 1H), 7.26 (dd, 1H), 3.65 (q, 2H), 1.94-1.90 (m, 2H), 1.76-1.72 (m, 2H), 1.21 (t, 3H).

**Ethyl-7-(1-cyancyclopropyl)-3-(ethylsulfonyl)imidazo[1,2-a]pyridin-2-carboxylat**

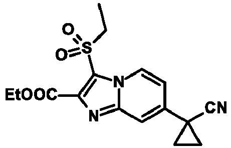

Zu einer Lösung von Ethyl-7-(1-cyancyclopropyl)-3-(ethylsulfanyl)imidazo[1,2-a]pyridin-2-carboxylat (18 g, 57.14 mmol) in Methanol (100 mL) und Wasser (100 mL) wurde Oxon (87.8 g, 285.7 mmol) gegeben. Das Reaktionsgemisch wurde 12 h bei Raumtemperatur gerührt. Das Lösungsmittel wurde im Vakuum entfernt und der Rückstand in Wasser aufgenommen. Die wässrige Phase wurde mit Dichlormethan extrahiert (3x). Die vereinte organische Phase wurde über Natriumsulfat getrocknet und im Vakuum einkonzentriert. Der Rückstand wurde mit Diethylether verrührt und der entstandene Feststoff abfiltriert. Das so erhaltene Rohprodukt wurde ohne weitere Aufreinigung im nächsten Schritt eingesetzt.

**Ethyl-7-(1-cyancyclopropyl)-3-(ethylsulfanyl)imidazo[1,2-a]pyridin-2-carboxylat**

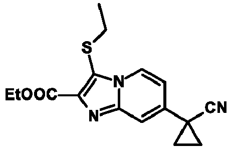

Zu einer Lösung von Ethyl-7-(1-cyancyclopropyl)-3-iodimidazo[1,2-a]pyridin-2-carboxylat (24 g, Rohprodukt) in 1,4-Dioxan (240 mL) wurde Diisopropylethylamin (18 mL, 126 mmol) gegeben und die Lösung für 5 min mit Argon entgast. Ethanthiol (7.02 mL, 94.5 mmol), Xantphos (4.36 g, 7.55 mmol) und Pd₂dba₃ (2.88 g, 3.15 mmol) wurden zugegeben und die Mischung für 10 min mit Argon entgast. Das Reaktionsgemisch wurde 2 h bei 120 °C gerührt. Nach Abkühlen auf Raumtemperatur wurde das Lösungsmittel im Vakuum entfernt. Der Rückstand wurde mittels Säulenchromatographie aufgereinigt.

MH⁺: 316; ¹H-NMR (400 MHz, CDCl₃) δ ppm: 8.53 (d, 1H), 7.55 (s, 1H), 6.97 (d, 1H), 4.49 (q, 2H), 2.94 (q, 2H), 1.87-1.84 (m, 2H), 1.52-1.42 (m, 5H), 1.21 (t, 3H).

**Ethyl-7-(1-cyancyclopropyl)-3-iodimidazo[1,2-a]pyridin-2-carboxylat**

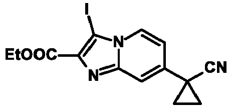

Zu einer Lösung von Ethyl-7-(1-cyancyclopropyl)imidazo[1,2-a]pyridin-2-carboxylat (22 g, Rohprodukt) in Acetonitril (220 mL) wurde portionsweise N-Iodsuccinimid (29.1 g, 129.4 mmol) zugegeben und das Reaktionsgemisch 4 h bei Raumtemperatur gerührt. Das Lösungsmittel wurde im Vakuum entfernt und der Rückstand in Wasser aufgenommen. Die wässrige Phase wurde mit Dichlormethan extrahiert (3x). Die vereinte organische Phase wurde über Natriumsulfat getrocknet und im Vakuum einkonzentriert. Der Rückstand wurde mit Diethylether verrührt und der entstandene Feststoff abfiltriert. Das so erhaltene Rohprodukt wurde ohne weitere Aufreinigung im nächsten Schritt eingesetzt.

¹H-NMR (400 MHz, CDCl₃) δ ppm: 8.24 (d, 1H), 7.54 (s, 1H), 6.98 (d, 1H), 4.46 (q, 2H), 1.88-1.82 (m, 2H), 1.52-1.43 (m, 5H).

**Ethyl-7-(1-cyancyclopropyl)imidazo[1,2-a]pyridin-2-carboxylat**

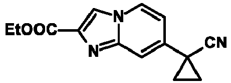

Zu einer Lösung von Ethyl-7-(cyanmethyl)imidazo[1,2-a]pyridin-2-carboxylat (20.2 g, Rohprodukt) in Acetonitril (200 mL) wurde Caesiumcarbonat (86.3 g, 264.6 mmol) und 1,2-Dibromethan (15.2 mL, 176.4 mmol) gegeben. Das Reaktionsgemisch wurde zunächst 1 h bei Raumtemperatur, anschließend 2 h bei 70 °C gerührt. Das Lösungsmittel wurde im Vakuum entfernt und der Rückstand in Wasser aufgenommen. Die wässrige Phase wurde mit Dichlormethan extrahiert (3x). Die vereinte organische Phase wurde über Natriumsulfat getrocknet und im Vakuum einkonzentriert. Der Rückstand wurde mit Diethylether verrührt und der entstandene Feststoff abfiltriert. Das so erhaltene Rohprodukt wurde ohne weitere Aufreinigung im nächsten Schritt eingesetzt.

**Ethyl-7-(cyanmethyl)imidazo[1,2-a]pyridin-2-carboxylat**

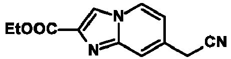

Zu einer Lösung von Ethyl-7-(chlormethyl)imidazo[1,2-a]pyridin-2-carboxylat (26 g Rohprodukt) in DMSO (260 mL) wurde NaCN (4.7 g, 95.9 mmol) gegeben. Das Reaktionsgemisch wurde 12 h bei Raumtemperatur gerührt. Das Reaktionsgemsich wurde mit eiskaltem Wasser verdünnt und mit Ethylacetat (2x) extrahiert. Die vereinte organische Phase wurde über Natriumsulfat getrocknet und im Vakuum einkonzentriert. Der Rückstand wurde mit Diethylether verrührt und der entstandene Feststoff abfiltriert. Das so erhaltene Rohprodukt wurde ohne weitere Aufreinigung im nächsten Schritt eingesetzt.

MH⁺: 230; ¹H-NMR (400 MHz, CDCl₃) δ ppm: 8.20 (s, 1H), 8.17 (d, 1H), 7.65 (d, 1H), 6.87 (dd, 1H), 4.47 (q, 2H), 3.82 (s, 2H), 1.44 (t, 3H).

**Ethyl-7-(chlormethyl)imidazo[1,2-a]pyridin-2-carboxylat**

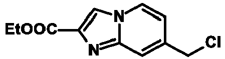

Zu einer Lösung von Ethyl-7-(hydroxymethyl)imidazo[1,2-a]pyridin-2-carboxylat (30.0 g, 136.36 mmol) in Dichlormethan (300 mL) wurde Triethylamin (59 mL, 409.08 mmol) und Methansulfonylchlorid (15.5 mL, 204.5 mmol) gegeben. Das Reaktionsgemisch wurde 2 h bei Raumtemperatur gerührt. Die Reaktionsmischung wurde mit Dichlormethan verdünnt und mit Wasser gewaschen. Die organische Phase wurde über Natriumsulfat getrocknet und das Lösungsmittel im Vakuum entfernt. Das Rohprodukt wurde ohne weitere Aufreinigung im nächsten Schritt eingesetzt.

MH⁺: 239; ¹H-NMR (400 MHz, CDCl₃) δ ppm: 8.18 (s, 1H), 8.14 (d, 1H), 7.63 (s, 1H), 6.93 (d, 1H), 4.60 (s, 2H), 4.43 (q, 2H), 1.42 (t, 3H).

**Ethyl-7-(hydroxymethyl)imidazo[1,2-a]pyridin-2-carboxylat**

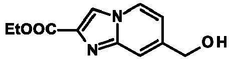

Zu einer Lösung von (2-Aminopyridin-4-yl)methanol (40.0 g, 322.5 mmol) in Ethanol (400 mL) wurde Ethyl-3-brom-2-oxopropanoat (50.3 mL, 387 mmol) und NaHCO₃ (54.2 g, 645 mmol) gegeben. Das Reaktionsgemisch wurde 3 h bei 70 °C gerührt und anschließend im Vakuum einkonzentriert. Das Rohprodukt wurde durch säulenchromatographische Aufreinigung gereinigt.

MH⁺: 221; ¹H-NMR (300 MHz, D₆-DMSO) δ ppm: 8.50-8.47 (m, 2H), 7.47 (s, 1H), 6.94-6.91 (m, 1H), 5.48 (t, 1H), 4.55 (d, 2H), 4.30 (q, 2H), 1.32 (t, 3H).

**2-[3-(Ethylsulfonyl)-5,6,7,8-tetrahydroimidazo[1,2-a]pyridin-2-yl]-3,5-dimethyl-6-(trifluormethyl)-3,5-dihydro-4H-imidazo[4,5-c]pyridin-4-on (I-03)**

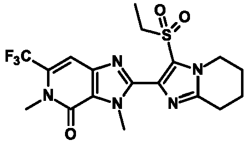

200 mg (0,77 mmol) 3-(Ethylsulfonyl)-5,6,7,8-tetrahydroimidazo[1,2-a]pyridin-2-carbonsäure, 324 mg (0,85 mmol) HATU und 300 mg (2,32 mmol) Diisoproylethylamin wurde in Tetrahydrofuran (10 mL) vorgelegt und 15 min bei Raumtemperatur gerrührt. Anschließend wurde 188 mg (0,85 mmol) 4-Amino-1-methyl-3-(methylamino)-6-(trifluormethyl)pyridin-2(1H)-on zugegeben und das Reaktionsgemisch 16 h bei Raumtemperatur gerührt. Das Reaktionsgemisch wurde unter vermindertem Druck vom Lösungsmittel befreit. Der Rückstand wurde in Dichlormethan aufgenommen und die organische Phase mit IN HCl (2x) und gesättigter Natriumchloridlösung gewaschen. Die organische Phase wurde über Natriumsulfat getrocknet, filtriert und unter vermindertem Druck vom Lösungsmittel befreit. Der Rückstand wurde in Essigsäure (15 mL) vorgelegt und bei 90 °C für 16 h gerührt. Der Eisessig wurde im Vakuum entfernt und der Rückstand in Dichlormethan aufgenommen. Die organische Phase wurde mit Wasser (3x) gewaschen, über Natriumsulfat getrocknet, filtriert und unter vermindertem Druck vom Lösungsmittel befreit. Das Rohprodukt wurde durch säulenchromatographische Aufreinigung über präparative HPLC gereinigt.

logP (sauer): 2,03; MH⁺: 444; ¹H-NMR(400 MHz, D₆-DMSO) δ ppm: 7.37 (s, 1H), 4.30-4.23 (m, 2H), 4.00 (s, 3H), 3.67-3.59 (m, 5H), 2.95-2.90 (m, 2H), 2.05-1.85 (m, 4H), 1.23 (t, 3H).

**3-(Ethylsulfonyl)-5,6,7,8-tetrahydroimidazo[1,2-a]pyridin-2-carbonsäure**

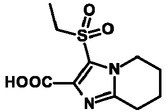

Eine Mischung von Ethyl-3-(ethylsulfanyl)-5,6,7,8-tetrahydroimidazo[1,2-a]pyridin-2-carboxylat (9.50 g, 33.2 mmol) und 15% wässrige HCl-Lösung wurde für 32 h bei 75 °C gerührt. Alles flüchtige wurde im Vakuum entfernt. Das Rohprodukt wurde ohne weitere Aufreinigung im nächsten Schritt eingesetzt.

MH⁺: 259; ¹H-NMR(400 MHz, D₆-DMSO) δ ppm: 4.20 (q, 2H), 3.58 (m, 2H), 2.87 (q, 2H), 1.94 (m, 2H), 1.83 (m, 2H), 1.22 (m, 3H).

**Ethyl-3-(ethylsulfonyl)-5,6,7,8-tetrahydroimidazo[1,2-a]pyridin-2-carboxylat**

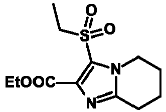

Eine Lösung von Ethyl-3-(ethylsulfanyl)-5,6,7,8-tetrahydroimidazo[1,2-a]pyridin-2-carboxylat (13.5 g, 53.1 mmol) in Dichlormethan (350 mL) wurde auf 0-5 °C abgekühlt und MCPBA (28.5 g, 165 mmol) portionsweise hinzugegeben. Das Reaktionsgemisch wurde langsam auf Raumtemperatur erhitzt und über Nacht gerührt. Die Mischung wurde auf eine 0 °C kalte wässrige Natriumhydrogencarbonat-Lösung gegossen und mit Dichlormethan (3x) extrahiert. Die vereinte organische Phase wurde mit gesättigter Natriumchlorid-Lösung gewaschen, über Natriumsulfat getrocknet, filtriert und unter vermindertem Druck vom Lösungsmittel befreit. Das Rohprodukt wurde durch säulenchromatographische Aufreinigung gereinigt.

**Ethyl-3-(ethylsulfanyl)-5,6,7,8-tetrahydroimidazo[1,2-a]pyridin-2-carboxylat**

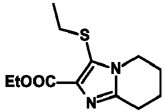

Ethanthiol (4.09 g, 65.9 mmol) wurde zu einer Lösung von Natriumhydrid (60% in Mineralöl, 2.20 g, 55.0 mmol) in Tetrahydrofuran (100 mL) getropft. Das Gemisch wurde für 10 min bei Raumtemperatur gerührt. Ethyl-3-brom-5,6,7,8-tetrahydroimidazo[1,2-a]pyridin-2-carboxylat (15.0 g, 54.9 mmol) wurde hinzugegeben und das Gemisch für 4 h unter Rückfluss erhitzt. Das Reaktionsgemisch wurde unter vermindertem Druck vom Lösungsmittel befreit und der Rückstand in Dichlormethan aufgenommen. Die organische Phase wurde mit Wasser gewaschen, über Natriumsulfat getrocknet, filtriert und unter vermindertem Druck vom Lösungsmittel befreit. Das so erhaltene Produkt wurde ohne weitere Aufreinigung eingesetzt.

**2-[7-(Cyclopropylsulfonyl)-3-(ethylsulfonyl)imidazo[1,2-a]pyridin-2-yl]-3,5-dimethyl-6-(trifluormethyl)-3,5-dihydro-4H-imidazo[4,5-c]pyridin-4-on (I-12)**

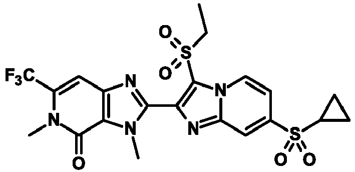

77,6 mg (0,15 mmol) 2-[7-Brom-3-(ethylsulfonyl)imidazo[1,2-a]pyridin-2-yl]-3,5-dimethyl-6-(trifluormethyl)-3,5-dihydro-4H-imidazo[4,5-c]pyridin-4-on wurde in DMSO (1 mL) vorgelegt. Unter Rühren wurden 5,2 mg (0,03 mmol) Kupferiodid und 76,7 mg (0,6 mmol) Natriumcyclopropansulfinat zugegeben. Das Gemisch wurde bei 120 °C für 6 h erhitzt. Die Reaktion wurde mit Ethylacetat verdünnt und mit Wasser (3 x) gewaschen. Die organische Phase wurde über Na₂SO₄ getrocknet, filtriert und unter vermindertem Druck vom Lösungsmittel befreit. Das Rohprodukt wurde durch säulenchromatographische Aufreinigung über präparative HPLC gereinigt.

logP (sauer): 2.59; MH⁺: 544; ¹H-NMR(300 MHz, D₆-DMSO) δ ppm: 9.23 (d, 1H), 8.47 (s, 1H), 7.76 (dd, 1H), 7.45 (s, 1H), 4.14 (s, 3H), 3.87 (q, 2H), 3.63 (s, 3H), 3.08-3.18 (m, 1H), 1.11-1.30 (m, 7H).

**2-(3-Ethylsulfonyl-7,7-dimethyl-6,8-dihydro-5H-imidazo[1,2-a]pyridin-2-yl)-3,5-dimethyl-6-(trifluormethyl)imidazo[4,5-c]pyridin-4-on (I-10)**

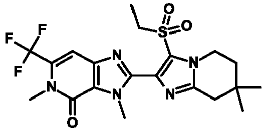

200 mg (0,43 mmol) 2-(3-Brom-7,7-dimethyl-6,8-dihydro-5H-imidazo[1,2-a]pyridin-2-yl)-3,5-dimethyl-6-(trifluormethyl)imidazo[4,5-c]pyridin-4-on, 8,3 mg (0,04 mmol) Kupferiodid und 507 mg (4,36 mmol) Natriumethylsulfinat wurden in 5,0 mL N,N-Dimethlyformamid gelöst. Das Reaktionsgemisch wurde 8 h bei 75 °C gerührt bis das Edukt vollständig umgesetzt war. Anschließend wurde das Gemisch mit Ethylacetat verdünnt. Die organische Phase wurde mit Wasser und gesättigter wässriger NaCl-Lösung gewaschen, über Na₂SO₄ getrocknet und am Rotatationsverdampfer eingeengt. Der erhaltene Rückstand wurde säulenchromatographisch mittels präparativer HPLC gereinigt.

logP (sauer): 2,66; M⁺: 471; ¹H-NMR (400 MHz, CDCl₃) δ ppm: 7.21 (s, 1H), 4.36 (t, 2H), 4.18 (s, 3H), 3.70 (br, 3H), 3.67 (q, 2H), 2.79 (s, 2H), 1.88 (t, 2H), 1.38 (t, 3H), 1.14 (s, 6H).

**2-(3-Brom-7,7-dimethyl-6,8-dihydro-5H-imidazo[1,2-a]pyridin-2-yl)-3,5-dimethyl-6-(trifluormethyl)imidazo[4,5-c]pyridin-4-on**

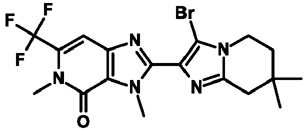

309 mg (1,13 mmol) 3-Brom-7,7-dimethyl-6,8-dihydro-5H-imidazo[1,2-a]pyridin-2-carbonsäure und 217 mg (1,13 mmol) *N*-(3-Dimethylaminopropyl)-N'-ethylcarbodiimid Hydrochlorid wurden in 7,0 mL Pyridin gelöst und für 30 min bei Raumtemperatur gerührt. Anschließend wurden 250 mg (1,13 mmol) 4-Amino-1-methyl-3-(methylamino)-6-(trifluormethyl)pyridin-2(1H)-on zugegeben und das Reaktionsgemisch für 16 h bei 120 °C gerührt. Das Lösungsmittel wurde anschließend unter vermindertem Druck entfernt, der Rückstand mit Ethylacetat aufgenommen und mit Wasser gewaschen. Die Phasen wurden getrennt und die organische Phase wurde am Rotationsverdampfer eingeengt. Der erhaltene Rückstand wurde in 15,0 mL Eisessig gelöst und das Reaktionsgemisch 16 h bei 100 °C gerührt. Anschließend wurde das Lösungsmittel entfernt und das Rohprodukt säulenchromatographisch an Kieselgel (Cyclohexan / Ethylacetat) gereinigt.

logP (sauer): 3,14; MH⁺: 471; ¹H-NMR (400 MHz, CDCl₃) δ ppm: 7.29 (s, 1H), 4.40 (s, 3H), 3.95 (t, 2H), 3.71 (s, 3H), 2.71 (s, 2H), 1.87 (t, 2H), 1.12 (s, 6H).

**3-Brom-7,7-dimethyl-6,8-dihydro-5H-imidazo[1,2-a]pyridin-2-carbonsäure**

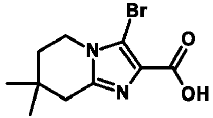

1,85 g (7,61 mmol) 7,7-Dimethyl-6,8-dihydro-5H-imidazo[1,2-a]pyridin-2-carbonsäure wurden in 30,0 mL N,N-Dimethlyformamid gelöst. Das Reaktionsgemisch wurde unter Eiskühlung mit 1,56 g (8,76 mmol) N-Bromsuccinimid versetzt und anschließend 16 h bei Raumtemperatur gerührt. Das Lösungsmittel wurde unter vermindertem Druck entfernt und der Rückstand säulenchromatographisch an Kieselgel (Dichlormethan / Methanol) gereinigt.

logP (sauer): 0,67; M⁺: 273; ¹H-NMR (400 MHz, D₆-DMSO) δ ppm: 3.93 (t, 2H), 2.68 (s, 2H), 1.83 (t, 2H), 1.03 (s, 6H). Das -COO**H** Proton ist im gemessenen Spektrum nicht sichtbar.

### Analytische Bestimmungen

Die nachstehend beschriebenen Durchführungen der analytischen Bestimmungen beziehen sich auf alle Angaben im gesamten Dokument, sofern die Durchführung der jeweiligen analytischen Bestimmung an der jeweiligen Textstelle nicht gesondert beschrieben ist.

### Massenspektrometrie

Die Bestimmung von [M+H]⁺ oder M⁻ mittels LC-MS unter sauren chromatographischen Bedingungen wurde mit 1 ml Ameisensäure pro Liter Acetonitril und 0.9 ml Ameisensäure pro Liter Millipore-Wasser als Eluenten durchgeführt. Es wurde die Säule Zorbax Eclipse Plus C18 50 mm ^{∗} 2.1 mm, 1.8 µm verwendet, bei einer Temperatur des Säulenofens von 55°C.

### Instrumente:

**LC-MS3**: Waters UPLC mit SQD2 Massenspektrometer und SampleManager Probenwechsler. Linearer Gradient 0.0 bis 1.70 Minuten von 10 % Acetonitril zu 95 % Acetonitril, von 1.70 bis 2.40 Minuten konstant 95 % Acetonitril, Fluss 0.85 ml/min.

**LC-MS6 und LC-MS7**: Agilent 1290 LC, Agilent MSD Massenspektrometer, HTS PAL Probenwechsler. Linearer Gradient 0.0 bis 1.80 Minuten von 10 % Acetonitril zu 95 % Acetonitril, von 1.80 bis 2.50 Minuten konstant 95 % Acetonitril, Fluss 1.0 ml/min).

Die Bestimmung von [M+H]⁺mittels LC-MS unter neutralen chromatographischen Bedingungen wurde mit Acetonitril und Millipore-Wasser mit 79 mg/l Ammoniumcarbonat als Eluenten durchgeführt.

### Instrumente:

**LC-MS4**: Waters IClass Acquity mit QDA Massenspektrometer und FTN Probenwechsler (Säule Waters Acquity 1.7 µm 50 mm ^{∗} 2.1 mm, Säulenofentemperatur 45°C). Linearer Gradient 0.0 bis 2.10 Minuten von 10 % Acetonitril zu 95 % Acetonitril, von 2.10 bis 3.00 Minuten konstant 95 % Acetonitril, Fluss 0.7 ml/min.

**LC-MS5**: Agilent 1100 LC System mit MSD Massenspektrometer und HTS PAL Probenwechsler (Säule: Zorbax XDB C18 1.8 µm 50 mm ^{∗} 4.6 mm, Säulenofentemperatur 55°C). Linearer Gradient 0.0 bis 4.25 Minuten von 10 % Acetonitril zu 95 % Acetonitril, von 4.25 bis 5.80 Minuten konstant 95 % Acetonitril, Fluss 2.0 ml/min.

Die Retentionzeit-Indizes wurden in allen Fällen aus einer Kalibrierungsmessung einer homologen Serie von geradkettigen Alkan-2-onen mit 3 bis 16 Kohlenstoffen bestimmt, wobei der Index des ersten Alkanons auf 300, der des letzten auf 1600 gesetzt und zwischen den Werten aufeinanderfolgender Alkanone linear interpoliert wurde.

### logP-Werte

Die Bestimmung der logP-Werte erfolgte gemäß EEC Directive 79/831 Annex V.A8 durch HPLC (High Performance Liquid Chromatography) an einer Phasenumkehrsäule (C18) mit Hilfe folgender Methoden:
[a] Der logP Wert wird durch LC-UV Messung im sauren Bereich bestimmt, mit 0.9 ml/l Ameisensäure in Wasser und 1.0 ml/l Ameisensäure in Acetonitril als Eluenten (linearer Gradient von 10% Acetonitrile bis 95% Acetonitril).
[b] Der logP Wert wird durch LC-UV Messung im neutralen Bereich bestimmt, mit 79 mg/l Ammoniumcarbonat in Wasser und Acetonitril als Eluenten (linearer Gradient von 10% Acetonitril bis 95% Acetonitril).

Die Kalibrierung wurde mit einer homologen Reihe geradkettiger Alkan-2-one (mit 3 bis 16 Kohlenstoffatomen) mit bekannten logP Werten durchgeführt. Die Werte zwischen aufeinanderfolgender Alkanone werden durch lineare Regression bestimmt.

### ¹H-NMR Spektren

Die Messungen der ¹H-NMR Spektren wurden mit einem Bruker **Avance III 400 MHz** Spektrometer, ausgestattet mit einem 1.7 mm TCI Probenkopf, mit Tetramethylsilan als Standard (0.00 ppm) von Lösungen in den Lösungsmitteln CD₃CN, CDCl₃ oder d₆-DMSO durchgeführt. Alternativ wurde ein **Bruker Avance III 600** MHz Spektrometer ausgestattet mit einem 5 mm CPNMP Probenkopf oder ein Bruker **Avance NEO 600** MHz Spektrometer ausgestattet mit einem 5 mm TCI Probenkopf für die Messungen verwendet. In der Regel wurden die Messungen bei einer Probenkopftemperatur von 298 K durchgeführt. Sofern andere Messtemperaturen verwendet wurden, wird dies gesondert vermerkt.

### NMR-Peaklisten-Verfahren

Die ¹H-NMR-Daten ausgewählter Beispiele werden in Form von ¹H-NMR-Peaklisten dargestellt. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispiels hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einer gedruckten Darstellung eines ¹H-NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren wird Tetramethylsilan genutzt oder die chemische Verschiebung des Lösungsmittels, falls die Probe kein Tetramethylsilan enthält. Daher können die ¹H-NMR-Peaklisten unter Umständen den Tetramethylsilan-Peak enthalten.

Die Listen der ¹H-NMR-Peaks sind äquivalent zu den klassischen ¹H-NMR-Darstellungen und enthalten somit gewöhnlich alle Peaks, die bei einer klassischen ¹H-NMR-Interpretation aufgeführt werden.

Darüber hinaus können sie wie klassische ¹H-NMR-Darstellungen Lösungsmittelsignale, Signale von Stereoisomeren der erfindungsgemäßen Verbindungen, die gegebenenfalls Gegenstand der Erfindung sind, und/oder Peaks von Verunreinigungen zeigen.

NMR-Lösungsmittelsignale, der Tetramethylsilan-Peak und das Wassersignal im jeweiligen Lösungsmittel sind von der relativen Intensitätskalibrierung ausgenommen, weil die dafür angegebenen Intensitätswerte sehr hoch sein können.

Die Peaks von (Stereo)Isomeren der erfindungsgemäßen Verbindungen und/oder Peaks von Verunreinigungen haben gewöhnlich im Durchschnitt eine geringere Intensität als die Peaks der erfindungsgemäßen Verbindungen (zum Beispiel bei einer Reinheit von >90%).

Solche Stereoisomere und/oder Verunreinigungen können typisch für das jeweilige Herstellungsverfahren sein. Ihre Peaks können somit dabei helfen, die Reproduktion eines Herstellungsverfahrens anhand von "Nebenprodukt-Fingerabdrücken" zu erkennen.

Einem Experten, der die Peaks der erfindungsgemäßen Verbindungen mit bekannten Verfahren (MestreC, ACD-Simulation, aber auch mit empirisch ausgewerteten Erwartungswerten) berechnet, kann je nach Bedarf die Peaks der erfindungsgemäßen Verbindungen identifizieren, wobei gegebenenfalls zusätzliche Intensitätsfilter eingesetzt werden. Diese Identifizierung ist äquivalent zur betreffenden Peak-Auflistung bei der klassischen 1H-NMR-Interpretation.

Das benutzte Lösungsmittel kann aus der JCAMP-Datei mit dem Parameter "solvent" ausgelesen werden, die Messfrequenz des Spektrometers mit "observe frequency" und das Spektrometermodell mit "spectrometer/data system".

¹³C-NMR-Daten werden analog zu den ¹H-NMR Daten als Peaklisten aus breitbandentkoppelten ¹³C-NMR-Spektren angegeben. Auch hier sind die NMR-Lösungsmittelsignale und Tetramethylsilan aus der relativen Intensitätskalibrierung herausgenommen, weil diese Signale sehr hohe Intensitätswerte haben können.

Weitere Details zu ¹H-NMR-Peaklisten können entnommen werden aus: "Citation of NMR Peaklist Data within Patent Applications" in der Research Disclosure Database Number 564025.

Die in Tabelle 1 aufgeführten Verbindungen wurden wie oben beschrieben oder analog dazu hergestellt.

**Tabelle 1:**

| **Beispiel** | **Struktur** | |
|---|---|---|
| I-01 | 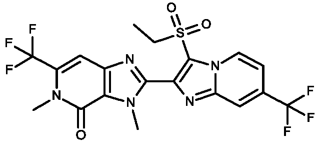 | I-01: ¹H-NMR(601.6 MHz, d₆-DMSO): δ= 9.2219 (1.4); 9.2096 (1.5); 8.5270 (1.9); 7.6476 (1.3); 7.6444 (1.3); 7.6353 (1.3); 7.6320 (1.3); 7.4385 (4.0); 4.1354 (16.0); 3.8902 (0.8); 3.8780 (3.0); 3.8657 (3.1); 3.8536 (0.9); 3.6314 (6.2); 3.3110 (13.6); 3.3069 (33.2); 2.6133 (0.4); 2.5225 (0.7); 2.5195 (0.9); 2.5164 (0.8); 2.5076 (20.3); 2.5046 (45.6); 2.5015 (64.2); 2.4984 (46.0); 2.4954 (21.2); 2.3857 (0.4); 1.2870 (3.3); 1.2747 (7.6); 1.2625 (3.3); 0.0052 (0.8); -0.0002 (27.8); -0.0057 (1.0) |
| 1-02 | 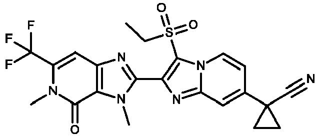 | 1-02: ¹H-NMR(400.2 MHz, d₆-DMSO): δ= 9.0279 (2.0); 9.0259 (1.9); 9.0094 (2.1); 9.0074 (2.0); 8.3145 (0.4); 7.8667 (2.2); 7.8648 (2.4); 7.8618 (2.4); 7.8597 (2.1); 7.4309 (4.1); 7.3598 (1.7); 7.3547 (1.6); 7.3413 (1.6); 7.3361 (1.6); 4.1242 (16.0); 3.8518 (0.9); 3.8333 (3.1); 3.8148 (3.2); 3.7965 (0.9); 3.6264 (6.6); 3.3199 (130.8); 2.6798 (0.5); 2.6753 (1.1); 2.6707 (1.5); 2.6661 (1.1); 2.6618 (0.5); 2.5242 (5.2); 2.5194 (8.4); 2.5108 (87.7); 2.5063 (170.1); 2.5017 (220.6); 2.4971 (160.1); 2.4926 (78.1); 2.3376 (0.5); 2.3331 (1.0); 2.3286 (1.4); 2.3240 (1.0); 2.3193 (0.5); 1.9614 (1.0); 1.9480 (2.5); 1.9403 (2.9); 1.9290 (1.4); 1.8068 (1.4); 1.7944 (2.8); 1.7872 (2.7); 1.7730 (0.9); 1.4450 (0.3); 1.4281 (0.3); 1.2653 (3.3); 1.2469 (7.8); 1.2284 (3.2); -0.0001 (1.2) |
| I-03 | 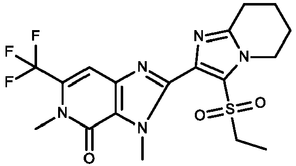 | 1-03: ¹H-NMR(400.2 MHz, d₆-DMSO): δ= 7.3648 (4.0); 5.7557 (2.3); 4.2743 (1.2); 4.2599 (2.3); 4.2448 (1.2); 3.9988 (16.0); 3.6535 (0.9); 3.6351 (3.2); 3.6165 (3.6); 3.6049 (6.7); 3.3275 (26.7); 2.9391 (1.2); 2.9232 (2.5); 2.9078 (1.2); 2.6754 (0.3); 2.6708 (0.4); 2.6660 (0.3); 2.5243 (1.4); 2.5195 (2.2); 2.5108 (27.1); 2.5064 (54.5); 2.5018 (71.4); 2.4972 (51.8); 2.4927 (25.3); 2.3286 (0.4); 2.3240 (0.3); 2.0201 (0.7); 2.0071 (1.2); 1.9956 (1.1); 1.9820 (0.5); 1.9267 (0.5); 1.9142 (1.2); 1.8993 (1.1); 1.8863 (0.8); 1.2468 (3.5); 1.2284 (7.9); 1.2099 (3.4); -0.0002 (1.3) |
| 1-04 | 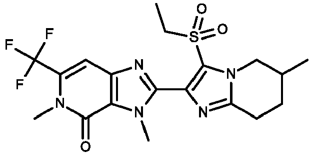 | 1-04: ¹H-NMR(400.2 MHz, CDCl3): δ= 7.2648 (8.0); 7.2201 (3.6); 5.3005 (5.7); 4.5885 (0.8); 4.5761 (0.8); 4.5550 (0.8); 4.5424 (0.8); 4.1773 (16.0); 3.7400 (1.0); 3.7066 (9.8); 3.6787 (1.7); 3.6589 (2.9); 3.6403 (3.0); 3.6218 (1.0); 3.1702 (0.4); 3.1653 (0.4); 3.1328 (0.5); 3.1265 (0.6); 3.1209 (0.6); 2.9900 (0.4); 2.9744 (0.5); 2.9619 (0.5); 2.9463 (0.8); 2.9306 (0.4); 2.9181 (0.4); 2.2025 (0.4); 2.1941 (0.4); 2.1853 (0.5); 2.1684 (0.4); 2.1119 (0.4); 2.1071 (0.5); 2.1042 (0.5); 2.0993 (0.5); 2.0887 (0.5); 2.0784 (0.5); 2.0734 (0.6); 2.0705 (0.6); 2.0657 (0.5); 1.6801 (0.4); 1.6658 (0.6); 1.6609 (0.4); 1.6517 (0.7); 1.6371 (0.4); 1.6323 (0.6); 1.6229 (0.4); 1.6182 (0.6); 1.4044 (4.5); 1.3859 (9.6); 1.3673 (4.4); 1.1992 (7.9); 1.1826 (7.6); 0.0706 (0.5); -0.0002 (7.3) |
| 1-05 | 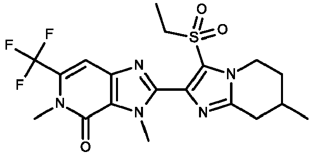 | 1-05: ¹H-NMR(400.2 MHz, CDCl3): δ= 7.2641 (9.6); 7.2056 (3.8); 5.3011 (1.3); 4.6201 (0.4); 4.6135 (0.5); 4.6069 (0.5); 4.5993 (0.4); 4.5854 (0.5); 4.5789 (0.6); 4.5723 (0.6); 4.5648 (0.5); 4.1783 (16.0); 4.1550 (0.4); 4.1379 (0.5); 4.1271 (0.6); 4.1037 (0.4); 4.0917 (0.3); 3.7026 (7.1); 3.6789 (1.7); 3.6587 (2.1); 3.6398 (1.8); 3.6217 (0.6); 3.6045 (0.4); 3.1815 (0.5); 3.1781 (0.5); 3.1692 (0.5); 3.1653 (0.6); 3.1385 (0.6); 3.1347 (0.6); 3.1258 (0.6); 3.1222 (0.6); 2.5844 (0.6); 2.5583 (0.8); 2.5413 (0.6); 2.5152 (0.7); 2.1640 (0.6); 2.1581 (0.6); 2.1535 (0.6); 2.1462 (0.5); 2.1342 (0.8); 2.1257 (1.0); 1.7572 (0.4); 1.7501 (0.3); 1.7434 (0.5); 1.7364 (0.3); 1.7290 (0.3); 1.7221 (0.4); 1.7152 (0.3); 1.7082 (0.4); 1.6114 (5.4); 1.3991 (3.5); 1.3806 (7.5); 1.3620 (3.4); 1.2042 (5.2); 1.1880 (5.2); 0.0079 (0.3); -0.0002 (9.4); -0.0083 (0.4) |
| 1-06 | 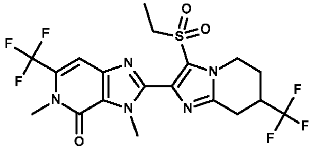 | 1-06: ¹H-NMR(400.2 MHz, CDCl3): δ= 7.2650 (7.2); 7.2100 (3.9); 4.7962 (0.3); 4.7892 (0.4); 4.7832 (0.4); 4.7757 (0.4); 4.7608 (0.4); 4.7538 (0.5); 4.7479 (0.5); 4.7405 (0.4); 4.2266 (0.4); 4.2139 (0.7); 4.2040 (16.0); 4.1913 (0.6); 4.1868 (0.5); 4.1624 (0.4); 4.1508 (0.3); 3.8085 (0.5); 3.7902 (0.7); 3.7733 (1.4); 3.7546 (1.4); 3.7330 (1.4); 3.7081 (6.8); 3.7058 (6.7); 3.6792 (0.5); 3.4093 (0.5); 3.4059 (0.5); 3.3957 (0.5); 3.3922 (0.5); 3.3662 (0.6); 3.3625 (0.6); 3.3524 (0.6); 3.3491 (0.6); 3.0823 (0.7); 3.0546 (0.8); 3.0391 (0.5); 3.0113 (0.7); 2.7715 (0.4); 2.4729 (0.4); 2.4662 (0.4); 2.4381 (0.5); 2.4314 (0.5); 2.1364 (0.5); 2.1225 (0.6); 2.1015 (0.5); 2.0875 (0.4); 1.6369 (4.2); 1.4090 (3.6); 1.3905 (7.7); 1.3719 (3.5); -0.0002 (8.6) |
| 1-07 | 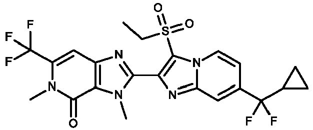 | 1-07: ¹H-NMR(400.2 MHz, d₆-DMSO): δ= 9.1348 (1.8); 9.1163 (1.9); 8.3155 (0.5); 8.1681 (2.4); 7.5433 (1.5); 7.5387 (1.6); 7.5248 (1.5); 7.5202 (1.6); 7.4435 (4.5); 4.1299 (16.0); 3.8755 (1.0); 3.8571 (3.3); 3.8387 (3.4); 3.8203 (1.0); 3.6304 (7.6); 3.3565 (0.4); 3.3239 (283.2); 2.6753 (1.2); 2.6707 (1.8); 2.6664 (1.3); 2.5241 (4.7); 2.5106 (101.7); 2.5063 (210.0); 2.5018 (280.9); 2.4973 (208.1); 2.4931 (105.0); 2.3332 (1.2); 2.3287 (1.7); 2.3241 (1.3); 1.9008 (0.4); 1.8849 (0.5); 1.8688 (0.6); 1.8521 (0.5); 1.8371 (0.4); 1.2847 (3.5); 1.2663 (8.0); 1.2478 (3.4); 0.7855 (9.6); 0.7695 (5.0); 0.1460 (0.5); 0.0079 (3.4); -0.0002 (108.0); -0.0083 (4.5); -0.1497 (0.5) |
| 1-08 | 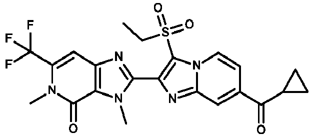 | 1-08: ¹H-NMR(400.2 MHz, d₆-DMSO): δ= 9.1269 (2.2); 9.1085 (2.4); 8.8502 (2.8); 8.3154 (0.4); 7.7610 (1.8); 7.7567 (1.8); 7.7427 (1.7); 7.7383 (1.7); 7.4485 (4.6); 4.1637 (16.0); 4.0921 (0.6); 3.9079 (1.1); 3.8896 (3.4); 3.8711 (3.4); 3.8524 (1.0); 3.6344 (8.1); 3.3245 (234.6); 3.1865 (0.6); 3.1804 (0.7); 3.1685 (1.1); 3.1561 (0.7); 3.1502 (0.6); 2.6753 (1.1); 2.6709 (1.5); 2.6663 (1.1); 2.5102 (90.2); 2.5063 (179.4); 2.5019 (236.5); 2.4975 (174.4); 2.3331 (1.1); 2.3287 (1.5); 2.3243 (1.1); 1.2906 (3.6); 1.2723 (8.1); 1.2620 (0.9); 1.2537 (3.6); 1.1703 (1.0); 1.1628 (2.8); 1.1422 (5.3); 1.1307 (3.3); 1.1224 (1.1); 0.7750 (0.4); 0.7586 (0.3); 0.1457 (0.4); 0.0078 (3.6); -0.0002 (97.6); -0.0083 (3.9); -0.1496 (0.4) |
| 1-09 | 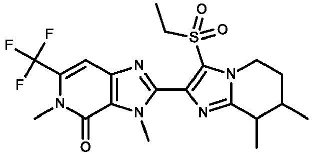 | 1-09: ¹H-NMR(400.2 MHz, CDCl3): δ= 7.2629 (10.9); 7.2023 (4.0); 4.4157 (0.6); 4.3970 (0.7); 4.3809 (1.1); 4.3654 (0.6); 4.3111 (0.6); 4.2947 (1.1); 4.2782 (0.8); 4.2599 (0.6); 4.2440 (0.3); 4.1804 (16.0); 3.7059 (7.2); 3.7041 (7.3); 3.6742 (1.0); 3.6556 (3.3); 3.6370 (3.5); 3.6185 (1.1); 3.2053 (0.7); 3.1920 (0.8); 3.1875 (0.8); 3.1743 (0.8); 2.6185 (1.0); 2.2722 (0.5); 2.2608 (0.6); 2.2548 (0.4); 2.2497 (0.5); 2.2444 (0.4); 2.0304 (0.7); 2.0248 (0.7); 2.0185 (1.3); 2.0134 (1.4); 1.9976 (1.1); 1.9801 (0.4); 1.6815 (0.5); 1.3951 (3.7); 1.3766 (7.9); 1.3606 (7.7); 1.3427 (6.5); 1.0608 (6.2); 1.0434 (6.0); 0.0078 (0.5); -0.0002 (13.4); -0.0084 (0.5) |
| 1-10 | 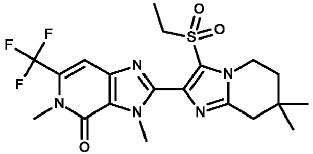 | 1-10: ¹H-NMR(400.2 MHz, CDCl3): δ= 7.2628 (8.5); 7.2067 (2.6); 4.3711 (1.1); 4.3552 (2.0); 4.3393 (1.1); 4.1840 (9.8); 3.7043 (5.8); 3.6857 (2.2); 3.6671 (2.2); 3.6485 (0.7); 2.7863 (4.0); 1.8920 (1.1); 1.8758 (1.9); 1.8601 (1.1); 1.6443 (1.4); 1.3988 (2.4); 1.3803 (5.1); 1.3616 (2.4); 1.1362 (16.0); -0.0002 (9.9); -0.0084 (0.4) |
| I-11 | 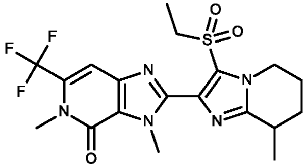 | I-11: ¹H-NMR(400.2 MHz, CDCl3): δ= 7.2629 (28.0); 7.2036 (3.6); 5.3013 (0.4); 4.5006 (0.5); 4.4884 (0.3); 4.4761 (0.4); 4.4661 (0.7); 4.4546 (0.4); 4.2252 (0.4); 4.2150 (0.4); 4.1993 (0.5); 4.1801 (16.0); 4.1668 (0.5); 4.1550 (0.3); 3.7057 (6.6); 3.7031 (6.5); 3.6884 (0.8); 3.6715 (1.3); 3.6529 (1.5); 3.6335 (1.5); 3.6148 (1.3); 3.5981 (0.6); 3.5797 (0.5); 3.0977 (0.5); 3.0819 (0.5); 2.2137 (0.3); 2.2082 (0.4); 2.1909 (0.8); 2.1794 (1.1); 2.1627 (0.5); 2.1446 (0.4); 2.0319 (0.4); 2.0186 (0.4); 2.0061 (0.4); 1.9889 (0.3); 1.6564 (0.3); 1.6335 (0.7); 1.6020 (0.8); 1.5835 (26.2); 1.4877 (6.4); 1.4702 (6.2); 1.4004 (3.4); 1.3818 (7.5); 1.3632 (3.3); 0.0079 (0.9); -0.0002 (31.6); -0.0084 (1.2) |
| 1-12 | 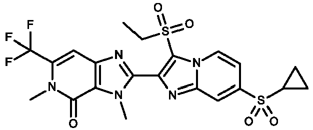 | 1-12: ¹H-NMR(400.2 MHz, d₆-DMSO): δ= 9.2431 (2.4); 9.2247 (2.5); 8.4737 (3.0); 8.4716 (3.0); 7.7767 (1.8); 7.7722 (1.8); 7.7583 (1.8); 7.7538 (1.8); 7.4507 (4.9); 4.1358 (16.0); 3.9057 (1.1); 3.8874 (3.5); 3.8689 (3.6); 3.8505 (1.1); 3.6328 (9.1); 3.3271 (179.6); 3.1443 (0.4); 3.1325 (0.7); 3.1246 (0.8); 3.1131 (1.4); 3.1014 (0.8); 3.0935 (0.8); 3.0815 (0.4); 2.6756 (0.8); 2.6714 (1.1); 2.6672 (0.8); 2.5067 (137.5); 2.5025 (175.0); 2.4983 (131.3); 2.3334 (0.8); 2.3292 (1.1); 2.3251 (0.8); 1.2998 (3.8); 1.2814 (8.8); 1.2631 (5.9); 1.2474 (1.1); 1.2194 (0.4); 1.1819 (0.8); 1.1705 (2.1); 1.1639 (2.2); 1.1509 (2.3); 1.1451 (1.9); 1.1316 (0.6); 0.1460 (0.8); 0.0074 (8.6); -0.0003 (158.8); -0.1495 (0.8) |

### Anwendungsbeispiele

### Boophilus microplus -Injektionstest

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: I-01.

### Ctenocephalides felis - Oraltest

### Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe *(Ctenocephalides felis)* werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: I-02.

Bei diesem Test zeigen z.B. die folgenden Verbindungen der Herstellungsbeispiele eine Wirkung von 95% bei einer Aufwandmenge von 100ppm: I-01.

### Diabrotica balteata - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Vorgequollene Weizenkörner (*Triticum aestivum*) werden in einer mit Agar und etwas Wasser gefüllten Multiwell-Platte für einen Tag inkubiert (5 Saatkörner pro Kavität). Die gekeimten Weizenkörner werden mit einer Wirkstoffzubereitung der gewünschten Konzentration gespritzt. Anschließend wird jede Kavität mit 10-20 Käferlarven von *Diabrotica balteata* infiziert.

Nach 7 Tagen wird die Wirkung in % bestimmt. Dabei bedeutet 100 %, dass alle Weizenpflanzen wie in der unbehandelten, nicht infizierten Kontrolle gewachsen sind; 0 % bedeutet, dass keine Weizenpflanze gewachsen ist.

Bei diesem Test zeigen z. B. die folgenden Verbindungen der Herstellungsbeispiele Wirkung von 100% bei einer Aufwandmenge von 160µg/Kavität: I-06, I-07, I-08, I-09, I-10, I-11, I-12.

### Meloidogyne incognita- Test

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita)* und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 100% bei einer Aufwandmenge von 20ppm: I-02.

Bei diesem Test zeigen z. B. die folgenden Verbindungen der Herstellungsbeispiele Wirkung von 90% bei einer Aufwandmenge von 20ppm: I-05.

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 100g/ha: I-02, I-04, I-05.

Bei diesem Test zeigen z. B. die folgenden Verbindungen der Herstellungsbeispiele Wirkung von 90% bei einer Aufwandmenge von 100g/ha: I-03, I-08, I-09, I-10, I-11.

### Myzus persicae - Oraltest

| | |
|---|---|
| Lösungsmittel: | 100 Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus *(Myzus persicae*)*,* die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 4ppm: I-01, I-02, I-03, I-04,I-05, I-06, I-07, I-08, I-09, I-10, I-11, I-12.

### Phaedon cochleariae - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 100g/ha: I-02.

### Spodoptera frugiperda - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 100g/ha: I-01, I-02, I-06, I-07, I-08, I-12.

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 100g/ha: I-09.

## Patentansprüche

1. Verbindungen der Formel (Ia) oder (Ib) 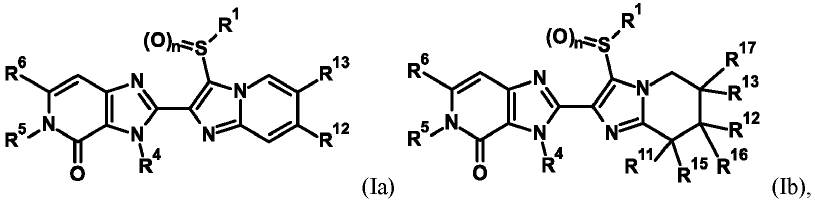 **dadurch gekennzeichnet, dass**
R¹ für (C₁-C₄)Alkyl steht,
R¹² für Wasserstoff, (C₁-C₄)Alkyl, Halogen, (C₃-C₆)Cycloalkyl-(C₁-C₆)halogenalkyl, (C₃-C₆)Cycloalkylsulfonyl, (C₃-C₆)Cycloalkylcarbonyl, (C₁-C₄)Halogenalkyl oder gegebenenfalls einfach durch Cyano substituiertes (C₃-C₆)Cycloalkyl steht,
R¹¹, R¹³, R¹⁶ unabhängig voneinander für Wasserstoff oder (C₁-C₄)Alkyl stehen,
R¹⁵, R¹⁷ hervorgehoben für Wasserstoff stehen,
wobei maximal zwei der Reste R¹¹, R¹², R¹³ oder R¹⁶ für einen Substituenten ungleich Wasserstoff stehen und
R⁴, R⁵ unabhängig voneinander für (C₁-C₄)Alkyl stehen,
R⁶ für (C₁-C₄)Halogenalkyl steht und
n für 2 steht.

2. Verbindungen der Formel (Ia) oder (Ib), gemäß Anspruch 1, **dadurch gekennzeichnet, dass**
R¹ für Ethyl steht,
R¹² für Wasserstoff, Cyanocyclopropyl, Methyl, Trifluormethyl, Cyclopropylcarbonyl, Cyclopropylsulfonyl oder Cyclopropyldifluormethyl steht,
R¹¹, R¹³, R¹⁶ unabhängig voneinander ür Wasserstoff oder Methyl stehen,
R¹⁵, R¹⁷ für Wasserstoff stehen,
wobei maximal zwei der Reste R¹¹, R¹², R¹³ oder R¹⁶ für einen Substituenten ungleich Wasserstoff stehen und
R⁴, R⁵ für Methyl stehen,
R⁶ für Trifluormethyl steht und
n für 2 steht.

3. Verbindungen der Formel (Ia') oder (Ib'), gemäß Anspruch 1, 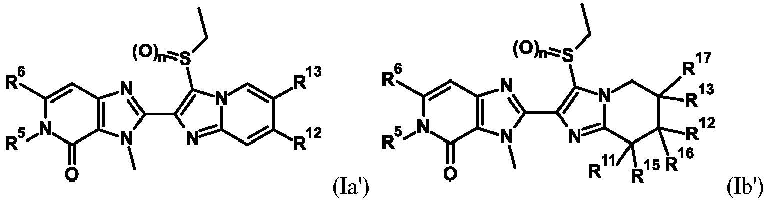 wobei R⁵, R⁶, R¹¹, R¹², R¹³, R¹⁵, R¹⁶, R¹⁷ und n gemäß einem der Ansprüche 1 bis 2 definiert sind.

4. Verbindungen der Formeln (Ia) oder (Ia'), gemäß einem der Ansprüche 1 bis 3, **dadurch gekennzeichnet, dass** R¹³ für Wasserstoff steht.

5. Agrochemische Formulierung enthaltend Verbindungen der Formel (Ia), (Ib), (Ia') oder (Ib') gemäß einem der Ansprüche 1 bis 4, sowie Streckmittel und/oder oberflächenaktive Substanzen.

6. Agrochemische Formulierung gemäß Anspruch 5 zusätzlich enthaltend einen weiteren agrochemischen Wirkstoff.

7. Verfahren zur Bekämpfung von tierischen Schädlingen **dadurch gekennzeichnet, dass** man eine Verbindung der Formel (Ia), (Ib), (Ia') oder (Ib') gemäß einem der Ansprüche 1 bis 4 oder eine agrochemische Formulierung gemäß einem der Ansprüche 5 oder 6 auf die tierischen Schädlinge und/oder ihren Lebensraum einwirken lässt, wobei Verfahren zur chirurgischen oder therapeutischen Behandlung des menschlichen oder tierischen Körpers und Diagnostizierverfahren, die am menschlichen oder tierischen Körper vorgenommen werden, ausgeschlossen sind.

8. Verwendung von Verbindungen der Formel (Ia), (Ib), (Ia') oder (Ib') gemäß einem der Ansprüche 1 bis 4 oder von agrochemischen Formulierungen gemäß einem der Ansprüche 5 oder 6 zur Bekämpfung von tierischen Schädlingen, wobei Verfahren zur chirurgischen oder therapeutischen Behandlung des menschlichen oder tierischen Körpers und Diagnostizierverfahren, die am menschlichen oder tierischen Körper vorgenommen werden, ausgeschlossen sind.

## Claims

1. Compound of the formula (Ia) or (Ib) 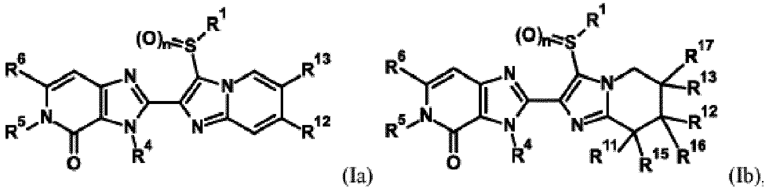 **characterized in that**
R¹ represents (C₁-C₄) -alkyl,
R¹² represents hydrogen, (C₁-C₄) -alkyl, halogen, (C₃-C₆) -cycloalkyl- (C₁-C₆) -haloalkyl, (C₃-C₆)-cycloalkylsulfonyl, (C₃-C₆)-cycloalkylcarbonyl, (C₁-C₄)-haloalkyl or (C₃-C₆)-cycloalkyl which is optionally monosubstituted by cyano,
R¹¹, R¹³, R¹⁶ independently of one another represent hydrogen or (C₁-C₄)-alkyl,
R¹⁵, R¹⁷ with emphasis represent hydrogen,
where at most two of the radicals R¹¹, R¹², R¹³ or R¹⁶ represent a substituent different from hydrogen and
R⁴, R⁵ independently of one another represent (C₁-C₄)-alkyl,
R⁶ represents (C₁-C₄)-haloalkyl and
n represents 2.

2. Compound of the formula (Ia) or (Ib) according to Claim 1, **characterized in that**
R¹ represents ethyl,
R¹² represents hydrogen, cyanocyclopropyl, methyl, trifluoromethyl, cyclopropylcarbonyl, cyclopropylsulfonyl or cyclopropyldifluoromethyl,
R¹¹, R¹³, R¹⁶ independently of one another represent hydrogen or methyl,
R¹⁵, R¹⁷ represent hydrogen,
where at most two of the radicals R¹¹, R¹², R¹³ or R¹⁶ represent a substituent different from hydrogen and
R⁴, R⁵ represent methyl,
R⁶ represents trifluoromethyl and
n represents 2.

3. Compound of the formula (Ia') or (Ib') according to Claim 1 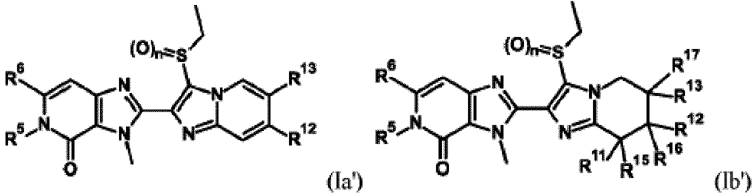 where R⁵, R⁶, R¹¹, R¹², R¹³, R¹⁵, R¹⁶, R¹⁷ and n are defined according to either of Claims 1 and 2.

4. Compound of the formula (Ia) or (Ia') according to any of Claims 1 to 3, **characterized in that** R¹³ represents hydrogen.

5. Agrochemical formulation comprising compounds of the formula (Ia), (Ib), (Ia') or (Ib') according to any of Claims 1 to 4 and extenders and/or surfactants.

6. Agrochemical formulation according to Claim 5, additionally comprising a further agrochemically active compound.

7. Method of controlling animal pests, **characterized in that** a compound of the formula (Ia), (Ib), (Ia') or (Ib') according to any of Claims 1 to 4 or an agrochemical formulation according to Claim 5 or 6 is allowed to act on the animal pests and/or their habitat, excluding methods of surgical or therapeutic treatment of the human or animal body and diagnostic methods performed on the human or animal body.

8. Use of compounds of the formula (Ia), (Ib), (Ia') or (Ib') according to any of Claims 1 to 4 or of agrochemical formulations according to Claim 5 or 6 for controlling animal pests, excluding methods of surgical or therapeutic treatment of the human or animal body and diagnostic methods performed on the human or animal body.

## Revendications

1. Composés de formule (Ia) ou (Ib) 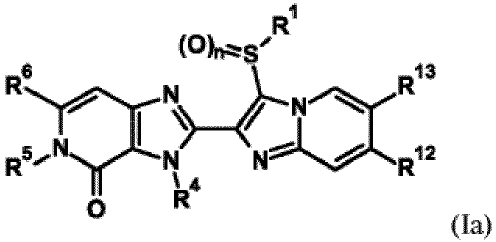 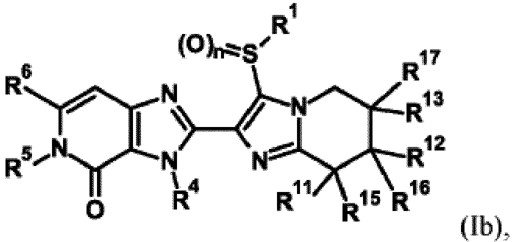 **caractérisés en ce que**
R¹ représente (C₁-C₄)alkyle,
R¹² représente hydrogène, (C₁-C₄)alkyle, halogène, (C₃-C₆) cycloalkyle-(C₁-C₆)halogénoalkyle, (C₃-C₆)cycloalkylsulfonyle, (C₃-C₆) cycloalkylcarbonyle, (C₁-C₄) halogénoalkyle ou (C₃-C₆)cycloalkyle éventuellement substitué une fois par cyano,
R¹¹, R¹³, R¹⁶ représentent indépendamment les uns des autres hydrogène ou (C₁-C₄)alkyle,
R¹⁵, R¹⁷ représentent de manière soulignée hydrogène,
dans lesquelles au maximum deux des radicaux R¹¹, R¹², R¹³ ou R¹⁶ représentent un substituant différent de l'hydrogène et
R⁴, R⁵ représentent indépendamment l'un de l'autre (C₁-C₄) alkyle,
R⁶ représente (C₁-C₄)halogénoalkyle et
n représente 2.

2. Composés de formule (Ia) ou (Ib), selon la revendication 1, **caractérisés en ce que**
R¹ représente éthyle,
R¹² représente hydrogène, cyanocyclopropyle, méthyle, trifluorométhyle, cyclopropylcarbonyle, cyclopropylsulfonyle ou cyclopropyldifluorométhyle,
R¹¹, R¹³, R¹⁶ représentent indépendamment les uns des autres hydrogène ou méthyle,
R¹⁵, R¹⁷ représentent hydrogène,
dans lesquelles au maximum deux des radicaux R¹¹, R¹², R¹³ ou R¹⁶ représentent un substituant différent de l'hydrogène et
R⁴, R⁵ représentent méthyle,
R⁶ représente trifluorométhyle et
n représente 2.

3. Composés de formule (Ia') ou (Ib'), selon la revendication 1, 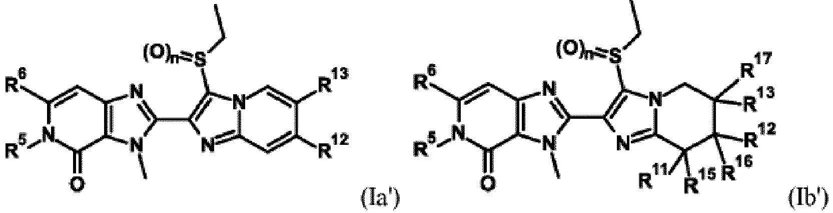 dans lesquelles R⁵, R⁶, R¹¹, R¹², R¹³, R¹⁵, R¹⁶, R¹⁷ et n sont définis selon l'une quelconque des revendication 1 et 2.

4. Composés de formule (Ia) ou (Ia'), selon l'une quelconque des revendications 1 à 3, **caractérisés en ce que** R¹³ représente hydrogène.

5. Formulation agrochimique contenant des composés de formule (Ia), (Ib), (Ia') ou (Ib') selon l'une quelconque des revendications 1 à 4, ainsi que des diluants et/ou des substances actives en surface.

6. Formulation agrochimique selon la revendication 5 contenant de plus un principe actif agrochimique supplémentaire.

7. Procédé pour la lutte contre des organismes nuisibles animaux, **caractérisé en ce qu'**on laisse agir un composé de formule (Ia), (Ib), (Ia') ou (Ib') selon l'une quelconque des revendications 1 à 4 ou une formulation agrochimique selon l'une quelconque des revendications 5 et 6 sur les organismes nuisibles animaux et/ou leur lieu de vie, dans lequel des procédés pour le traitement chirurgical ou thérapeutique du corps humain ou animal et des procédés de diagnostic, qui sont entrepris sur le corps humain ou animal, sont exclus.

8. Utilisation de composés de formule (Ia), (Ib), (Ia') ou (Ib') selon l'une quelconque des revendications 1 à 4 ou de formulations agrochimiques selon l'une quelconque des revendications 5 et 6 pour la lutte contre des organismes nuisibles animaux, dans laquelle des procédés pour le traitement chirurgical ou thérapeutique du corps humain ou animal et des procédés de diagnostic, qui sont entrepris sur le corps humain ou animal, sont exclus.
